# EUROPEAN PATENT SPECIFICATION

(11) **EP 3 962 564 B1**
(45) Date of publication and mention of the grant of the patent: **05.11.2025**
(21) Application number: 19931087.1
(22) Date of filing: 24.05.2019
(51) Int. Cl.: A61M 25/01, A61B 17/34

(54) **ARTICULATED STIFFENING CANNULAS AND ACCESS SETS**
GELENKIGE VERSTEIFUNGSKANÜLEN UND ZUGANGSSETS
CANULES DE RAIDISSEMENT ARTICULÉES ET ENSEMBLES D'ACCÈS

(43) Date of publication of application: 09.03.2022
(73) Proprietor: Becton, Dickinson and Company, Franklin Lakes, NJ 07417-1880 (US)
(72) Inventor: SUN, Xiaowen, Shanghai 200030 (CN); HE, Zhixiu, Shanghai 201100 (CN); ZENG, Xi, Suzhou, Jiangsu 215008 (CN); YI, Peng, Shanghai 201102 (CN)
(74) Representative: Hoffmann Eitle
(86) International application number: PCT/CN2019/088334
(87) International publication number: WO 2020/237426

(56) References cited:
- CN-A- 107 073 723
- CN-A- 107 374 702
- CN-A- 108 618 739
- CN-A- 108 618 739
- US-A- 5 848 986
- US-A1- 2002 082 584
- US-A1- 2006 259 061
- US-A1- 2008 255 423
- US-A1- 2011 137 125
- US-A1- 2016 331 443
- US-A1- 2017 049 511
- US-A1- 2018 311 470
- US-A1- 2019 117 937
- US-B1- 8 287 481

## Description

### BACKGROUND

In a healthy person, blood flowing from the stomach, esophagus, or intestines first flows through the liver. In an unhealthy person having, for example, liver damage, there can be blood flow-restricting blockages in the liver such that blood cannot easily flow therethrough. Such a condition is known as portal hypertension. Common causes of portal hypertension include alcohol abuse, too much iron in the liver (e.g., hemochromatosis), hepatitis B, hepatitis C, or blood clots in a vein that flows from the liver to the heart. When portal hypertension occurs, the blood flow-restricting blockages can elevate pressure in the portal vein causing it to rupture and seriously bleed. A person with portal hypertension can also have bleeding from the veins of the stomach, esophagus, or intestines (e.g., variceal bleeding), a buildup of fluid in the belly (e.g., ascites), or a buildup of fluid in the chest (e.g., hydrothorax).

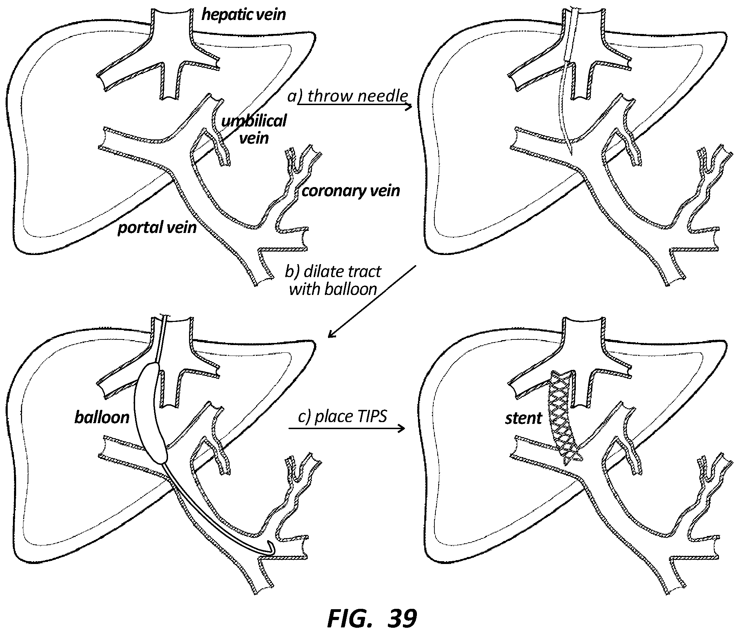

Portal hypertension is often treated by way of a percutaneous procedure involving placement of a transjugular intrahepatic portosystemic shunt ("TIPS") between the hepatic vein and the portal vein as shown in FIG. 39 to establish blood flow through the liver. Placement of a portosystemic shunt between the right hepatic vein and the right portal vein is generally preferred, and transjugular liver access sets currently available are generally dedicated to this end. For example, bent or curved stiffening cannulas are often provided in the foregoing transjugular liver access sets to support an anterior needle throw from the right hepatic vein to the right portal vein. Being that the stiffening cannulas are already bent or curved, the stiffening cannulas do not easily accommodate anatomical variations in patients or different modes of access within the liver such as from the middle hepatic vein to either the left or right portal vein, which respectively requires either an anterior or posterior needle throw, or the left hepatic vein to the left portal vein, which requires a posterior needle throw. In view of the foregoing, the transjugular liver access sets currently available can inadvertently contribute to prolonged procedures and decreased success rates for patients having anatomical variations or needing different modes of access within the liver.

US 5 848 986 A discloses a trans-urethal needle device slidably mounted in a steerable guide tube. US 2017/049511 A1 discloses an introducer sheath, a steerable guiding sheath and a crossing catheter for accessing a hepatic vein.

Disclosed herein are articulated stiffening cannulas, access sets, and methods thereof that address at least the forgoing shortcomings.

### SUMMARY

The invention is defined by claims 1 and 12. Further embodiments of the invention are defined by the dependent claims.

Disclosed herein is an articulated stiffening cannula including, a cannula tube, a compound hub disposed about a proximal-end portion of the cannula tube, and an articulation mechanism controlled by at least a rotatable element of the compound hub. The cannula tube includes a flexible hinge along a distal length of the cannula tube. The cannula tube is configured to articulate at the flexible hinge for adjusting a cannula-tube angle between a distal-end portion of the cannula tube and a proximal length of the cannula tube. The articulation mechanism includes a single control line or a number of control lines extending from the compound hub, through the cannula tube beyond the flexible hinge, and to the distal-end portion of the cannula tube for adjusting the cannula-tube angle. The stiffening cannula is configured to stiffen a catheter assembly in support of a needle throw from the catheter assembly at a chosen cannula-tube angle.

In some embodiments, the cannula tube includes a number of transverse cutouts in the cannula tube. The flexible hinge is formed at least in part by the cutouts.

In some embodiments, the cannula tube includes a needle lumen longitudinally extending through the cannula tube. The needle lumen is configured for disposing at least a 5-Fr catheter-and-needle assembly therein.

The single control line or each control line of the control lines is disposed in a separate, dedicated control-line lumen longitudinally extending through the cannula tube.

In some embodiments, the rotatable element is configured to draw the single control line or a first control line of the control lines into the compound hub upon rotating the rotatable element in a first direction. The rotatable element is also configured to let the single control line or the first control line out of the compound hub upon rotating the rotatable element in a second direction opposite the first direction.

In some embodiments, the first direction of the rotatable element increases the cannula-tube angle, and the second direction of the rotatable element decreases the cannula-tube angle.

In some embodiments, the rotatable element is configured to let a second control line of the control lines out of the compound hub upon rotating the rotatable element in the second direction. The rotatable element is also configured to draw the second control line into the compound hub upon rotating the rotatable element in the first direction.

In some embodiments, the cannula-tube angle ranges from about 0° to about 35°.

Also disclosed herein is an articulated stiffening cannula including, a cannula tube, a compound hub disposed about a proximal-end portion of the cannula tube, and an articulation mechanism controlled by at least a rotatable hub of the compound hub. The cannula tube includes a flexible hinge along a distal length of the cannula tube. The cannula tube is configured to articulate at the flexible hinge for adjusting a cannula-tube angle between a distal-end portion of the cannula tube and a proximal length of the cannula tube. The compound hub includes a fixed hub and the rotatable hub. The fixed hub is fixedly coupled to the cannula tube, and the rotatable hub is configured to rotate relative to the fixed hub. The articulation mechanism includes a single control line or a pair of control lines extending from the compound hub, through the cannula tube beyond the flexible hinge, and to the distal-end portion of the cannula tube for adjusting the cannula-tube angle. The stiffening cannula is configured to stiffen a catheter assembly in support of a needle throw from the catheter assembly at a chosen cannula-tube angle.

In some embodiments, the cannula tube is stainless steel or nitinol. The cannula tube includes a number of transverse cutouts filled with a pliable material. The flexible hinge is formed at least in part by the pliable material-filled cutouts.

In some embodiments, the cutouts include a first set of cutouts along a first side of the cannula tube and a second set of cutouts along a second side of the cannula tube opposite the first side of the cannula tube. The first set of cutouts is configured for compression when the cannula tube is articulated at the flexible hinge. The second set of cutouts is configured for tension when the cannula tube is articulated at the flexible hinge.

In some embodiments, both the control lines of the pair of control lines are disposed in a dedicated control-line lumen extending through the cannula tube alongside a needle lumen of the cannula tube.

In some embodiments, each control line of the pair of control lines is disposed in a separate, dedicated control-line lumen extending through the cannula tube alongside a needle lumen of the cannula tube.

In some embodiments, a proximal-end portion of a first control line of the pair of control lines is coupled to the rotatable hub, and a distal-end portion of the first control line is attached to the distal-end portion of the cannula tube. The rotatable hub is configured to draw the first control line into the compound hub upon rotating the rotatable hub in a first direction. The rotatable hub is also configured to let the first control line out of the compound hub upon rotating the rotatable hub in a second direction opposite the first direction.

In some embodiments, the first direction of the rotatable hub increases the cannula-tube angle, and the second direction of the rotatable hub decreases the cannula-tube angle.

In some embodiments, a proximal-end portion of a second control line of the pair of control lines is also coupled to the rotatable hub, and a distal-end portion of the second control line is also attached to the distal-end portion of the cannula tube. The rotatable hub is configured to let the second control line out of the compound hub upon rotating the rotatable hub in the second direction. The rotatable hub is configured to draw the second control line into the compound hub upon rotating the rotatable hub in the first direction.

In some embodiments, the rotatable hub is rotatably coupled to the cannula tube and disposed between the fixed hub and a fixed endpiece of the compound hub fixedly coupled to the cannula tube. The rotatable hub is engaged with the fixed hub in a Hirth or curvic coupling and held against the fixed hub by a compression spring between the rotatable hub and the fixed endpiece.

In some embodiments, the Hirth or curvic coupling is configured to hold the cannula tube at the chosen cannula-tube angle in combination with the compression spring.

In some embodiments, the cannula tube includes a cannula-tube core having one or more tubular layers disposed thereover. The cannula tube includes a number of transverse cutouts in at least the one or more tubular layers. The flexible hinge is formed at least in part by the cutouts.

In some embodiments, the single control line is disposed in a dedicated control-line lumen extending through the cannula tube alongside a needle lumen of the cannula tube.

In some embodiments, a proximal-end portion of the single control line is coupled to the rotatable hub, and a distal-end portion of the single control line is attached to the distal-end portion of the cannula tube. The rotatable hub is configured to draw the single control line into the compound hub upon rotating the rotatable hub in a first direction. The rotatable hub is also configured to let the single control line out of the compound hub upon rotating the rotatable hub in a second direction opposite the first direction.

In some embodiments, the first direction of the rotatable hub increases the cannula-tube angle, and the second direction of the rotatable hub decreases the cannula-tube angle.

In some embodiments, the rotatable hub is rotatably coupled to the cannula tube and disposed in a bore in a proximal-end portion of the fixed hub.

In some embodiments, the rotatable hub includes a threaded bore concentric with the bore in the fixed hub. The threaded bore is configured to drive a threaded plug proximally, away from the fixed hub, to increase the cannula-tube angle or distally, toward the fixed hub to decrease the cannula-tube angle.

Also disclosed is an articulated stiffening cannula including, in some embodiments, a cannula tube, a compound hub having a body disposed about a proximal-end portion of the cannula tube, and an articulation mechanism controlled by at least a rotatable knob of the compound hub. The cannula tube includes a flexible hinge along a distal length of the cannula tube. The cannula tube is configured to articulate at the flexible hinge for adjusting a cannula-tube angle between a distal-end portion of the cannula tube and a proximal length of the cannula tube. The articulation mechanism includes a leadscrew controlled by the rotatable knob. The articulation mechanism includes a pair of tensioned control lines extending from the compound hub, through the cannula tube beyond the flexible hinge, and to the distal-end portion of the cannula tube for adjusting the cannula-tube angle. The stiffening cannula is configured to stiffen a catheter assembly in support of a needle throw from the catheter assembly at a chosen cannula-tube angle.

In some embodiments, the cannula tube includes a cannula-tube core having one or more tubular layers disposed thereover. The cannula tube includes a number of transverse cutouts in at least the one or more tubular layers. The flexible hinge is formed at least in part by the cutouts.

In some embodiments, each control line of the pair of control lines is disposed in a separate, dedicated control-line lumen extending through the cannula tube alongside a needle lumen of the cannula tube.

In some embodiments, a proximal-end portion of a first control line of the pair of control lines is mounted on a first control-line mount, and a proximal-end portion of a second control line of the pair of control lines is mounted on a second control-line mount. Each mount of the first control-line mount and the second control-line mount is coupled to a pair of control arms. The pair of control arms are coupled to a leadscrew-nut block by way of a follower of the leadscrew-nut block.

In some embodiments, a distal-end portion of each of the first control line and the second control line is attached to a location opposite the other at the distal-end portion of the cannula tube. The articulation mechanism is configured to draw the first control line into the compound hub upon rotating the rotatable knob in a first direction. The articulation mechanism is also configured to let the second control line out of the compound hub upon rotating the rotatable knob in the first direction.

In some embodiments, the articulation mechanism is configured to let the first control line out of the compound hub upon rotating the rotatable knob in a second direction opposite the first direction. The articulation mechanism is also configured draw the second control line into the compound hub upon rotating the rotatable knob in the second direction.

In some embodiments, the first direction of the rotatable knob increases the cannula-tube angle, and the second direction of the rotatable knob decreases the cannula-tube angle.

In some embodiments, the compound hub includes markings on the body of the compound hub indicating the first direction for increasing the cannula-tube angle and the second direction for decreasing the cannula-tube angle.

In some embodiments, the compound hub includes a window disposed in a window opening of the body of the compound hub. The window includes graduations thereon for following an indictor on a window-facing side of the leadscrew-nut block behind the graduations of the window when the cannula-tube angle is changed.

Also disclosed herein is an articulated stiffening cannula including, in some embodiments, a cannula tube, a compound hub having a body disposed about a proximal-end portion of the cannula tube, and an articulation mechanism controlled by a dial. The cannula tube includes a flexible hinge along a distal length of the cannula tube. The cannula tube is configured to articulate at the flexible hinge for adjusting a cannula-tube angle between a distal-end portion of the cannula tube and a proximal length of the cannula tube. The articulation mechanism includes a pair of opposing extension tabs coupled to the dial, which extend from arcuate slots in the body of the compound hub for controlling the dial. The articulation mechanism includes a pair of tensioned control lines extending from the compound hub, through the cannula tube beyond the flexible hinge, and to the distal-end portion of the cannula tube for adjusting the cannula-tube angle. The stiffening cannula is configured to stiffen a catheter assembly in support of a needle throw from the catheter assembly at a chosen cannula-tube angle.

In some embodiments, the cannula tube includes a cannula-tube core having one or more tubular layers disposed thereover. The cannula tube includes a number of transverse cutouts in at least the one or more tubular layers. The flexible hinge is formed at least in part by the cutouts.

In some embodiments, the cutouts include a first set of cutouts along a first side of the cannula tube and a second set of cutouts along a second side of the cannula tube opposite the first side of the cannula. The first set of cutouts are staggered with the second set of cutouts.

In some embodiments, each control line of the pair of control lines is disposed in a separate, dedicated control-line lumen extending through the cannula tube alongside a needle lumen of the cannula tube.

In some embodiments, a proximal-end portion of a first control line of the pair of control lines is attached to a first control-line pin disposed in an inner annulus of the dial, and a proximal-end portion of a second control line of the pair of control lines is attached to a second control-line pin disposed in the inner annulus of the dial with the cannula tube between the first control-line pin and the second control-line pin.

In some embodiments, each pin of the first control-line pin and the second control-line pin is configured to subtend a same angle as a corresponding extension tab of the extension tabs when the dial is rotated. The corresponding extension tab of the first control-line pin or the second control-line pin tab is on a same side of the cannula tube as the first control-line pin or the second control-line pin.

In some embodiments, a distal-end portion of each of the first control line and the second control line is coupled to a location opposite the other at the distal-end portion of the cannula tube. The articulation mechanism is configured to draw the first control line into the compound hub upon rotating the dial in a first direction. The articulation mechanism is also configured to let the second control line out of the compound hub upon rotating the dial in the first direction.

In some embodiments, the articulation mechanism is configured to let the first control line out of the compound hub upon rotating the dial in a second direction opposite the first direction. The articulation mechanism is also configured to draw the second control line into the compound hub upon rotating the dial in the second direction.

In some embodiments, the first direction of the dial increases the cannula-tube angle and the second direction of the dial decreases the cannula-tube angle.

In some embodiments, the dial includes a pair of arcuate tracks in the dial. A first track of the pair of tracks is disposed in an outer annulus of the dial. A second track of the pair of tracks is disposed in the outer annulus of the dial. The cannula tube is between the first track and the second track.

In some embodiments, each track of the first track and the second track includes a number of wells configured to accept a corresponding spring-loaded plunger disposed therein. The corresponding plunger of the first track or the second track is on a same side of the cannula tube as the first track or the second track.

In some embodiments, the plunger corresponding to the first track or the second track makes audible clicks when the dial is rotated. Each audible click of the audible clicks corresponds to disposal of the plunger in a well of the wells.

In some embodiments, each well of the wells in the first track or the second track is a predetermined arcuate distance from another well in the first track or the second track. The arcuate distance corresponds to a stepwise change in the cannula-tube angle.

Also disclosed herein is an access set including, in some embodiments, an introducer sheath, an articulated stiffening cannula, a catheter, and a needle. The stiffening cannula includes a cannula tube, a compound hub disposed about a proximal-end portion of the cannula tube, and an articulation mechanism controlled by at least a rotatable element of the compound hub. The cannula tube has a flexible hinge along a distal length of the cannula tube. The cannula tube is configured to articulate at the flexible hinge for adjusting a cannula-tube angle between a distal-end portion of the cannula tube and a proximal length of the cannula tube. The articulation mechanism includes a single control line or a number of control lines extending from the compound hub, through the cannula tube beyond the flexible hinge, and to the distal-end portion of the cannula tube for adjusting the cannula-tube angle. The stiffening cannula is configured to stiffen at least a catheter-and-needle assembly in support of a needle throw at a chosen cannula-tube angle from the catheter-and-needle assembly when disposed in the stiffening cannula. The catheter-and-needle assembly includes the needle disposed in the catheter.

In some embodiments, the cannula tube includes a needle lumen longitudinally extending through the cannula tube configured for disposing at least a 5-Fr catheter-and-needle assembly therein.

Also disclosed herein is a method of an articulated stiffening cannula including, in some embodiments, inserting the stiffening cannula into an introducer sheath positioned in a distal portion of a hepatic vein, the stiffening cannula including a cannula tube having a flexible hinge along a distal length of the cannula tube for adjusting a cannula-tube angle between a distal-end portion of the cannula tube and a proximal length of the cannula tube; inserting a catheter-and-needle assembly into the stiffening cannula, the catheter-and-needle assembly including a needle disposed in a catheter, thereby stiffening at least the catheter-and-needle assembly; articulating the stiffening cannula by rotating a rotatable element of a compound hub of the stiffening cannula either before or after inserting the catheter-and-needle assembly; choosing the cannula-tube angle for a needle throw from the hepatic vein to a portal vein; and throwing the needle from the hepatic vein to the portal vein.

In some embodiments, throwing the needle from the hepatic vein to the portal vein includes throwing the needle from a right hepatic vein to a right portal vein with an anterior needle throw in accordance with the cannula-tube angle chosen for the needle throw.

In some embodiments, throwing the needle from the hepatic vein to the portal vein includes throwing the needle from a middle hepatic vein to either a left portal vein or a right portal vein respectively with either an anterior needle throw or a posterior needle throw in accordance with the cannula-tube angle chosen for the needle throw.

In some embodiments, throwing the needle from the hepatic vein to the portal vein includes throwing the needle from a left hepatic vein to a left portal vein with a posterior needle throw in accordance with the cannula-tube angle chosen for the needle throw.

These and other features of the concepts provided herein will become more apparent to those of skill in the art in view of the accompanying drawings and following description, which disclose particular embodiments of such concepts in greater detail.

### DRAWINGS

FIG. 1 illustrates a first articulated stiffening cannula, in accordance with some embodiments.
FIG. 2 illustrates a cannula tube of the first stiffening cannula adjusted in an arbitrary cannula-tube angle, in accordance with some embodiments.
FIG. 3 illustrates a first set of a number of cutouts along a first side of the cannula tube of the first stiffening cannula, in accordance with some embodiments.
FIG. 4 illustrates a second set of the cutouts along a second side of the cannula tube of the first stiffening cannula, in accordance with some embodiments.
FIG. 5 illustrates a longitudinal cross section of the cannula tube of the first stiffening cannula, in accordance with some embodiments.
FIG. 6 illustrates a transverse cross section of the cannula tube of the first stiffening cannula, in accordance with some embodiments.
FIG. 7 illustrates a transverse cross section of the cannula tube of the first stiffening cannula, in accordance with some embodiments.
FIG. 8 illustrates a transverse cross section of the cannula tube of the first stiffening cannula, in accordance with some embodiments.
FIG. 9 illustrates a compound hub of the first stiffening cannula, in accordance with some embodiments.
FIG. 10 illustrates a longitudinal cross section of the compound hub of the first stiffening cannula, in accordance with some embodiments.
FIG. 11 illustrates a transverse cross section of the compound hub of the first stiffening cannula, in accordance with some embodiments.
FIG. 12 illustrates a transverse cross section of the compound hub of the first stiffening cannula, in accordance with some embodiments.
FIG. 13 illustrates a second articulated stiffening cannula, in accordance with some embodiments.
FIG. 14 illustrates a compound hub of the second stiffening cannula, in accordance with some embodiments.
FIG. 15 illustrates a longitudinal cross section of the compound hub of the second stiffening cannula, in accordance with some embodiments.
FIG. 16 illustrates a longitudinal cross section of the compound hub of the second stiffening cannula, in accordance with some embodiments.
FIG. 17 illustrates a side of a cannula tube of the second stiffening cannula without cutouts, in accordance with some embodiments.
FIG. 18 illustrates a number of cutouts along the side of a cannula tube of the second stiffening cannula, in accordance with some embodiments.
FIG. 19 illustrates a transverse cross section of the cannula tube of the second stiffening cannula, in accordance with some embodiments.
FIG. 20 illustrates a first access set including a third articulated stiffening cannula, in accordance with some embodiments.
FIG. 21 illustrates a compound hub of the third stiffening cannula, in accordance with some embodiments.
FIG. 22 illustrates a longitudinal cross section of the compound hub of the third stiffening cannula, in accordance with some embodiments.
FIG. 23 illustrates a longitudinal cross section of the compound hub of the third stiffening cannula, in accordance with some embodiments.
FIG. 24 illustrates a transverse cross section of a cannula tube of the third stiffening cannula, in accordance with some embodiments.
FIG. 25 illustrates the cannula tube of the third stiffening cannula adjusted in an arbitrary cannula-tube angle, in accordance with some embodiments.
FIG. 26 illustrates markings on the compound hub of the third stiffening cannula for increasing the cannula-tube angle, in accordance with some embodiments.
FIG. 27 illustrates a window of the compound hub of the third stiffening cannula having cannula-tube-angle graduations, in accordance with some embodiments.
FIG. 28 illustrates the window of the compound hub of the third stiffening cannula having the cannula-tube-angle graduations, in accordance with some embodiments.
FIG. 29 illustrates a second access set including a fourth articulated stiffening cannula, in accordance with some embodiments.
FIG. 30 illustrates the fourth stiffening cannula, in accordance with some embodiments.
FIG. 31 illustrates the compound hub of the fourth stiffening cannula, in accordance with some embodiments.
FIG. 32 illustrates a first plurality of cutouts of a cannula tube of the fourth stiffening cannula, in accordance with some embodiments.
FIG. 33 illustrates a second plurality of cutouts of the cannula tube of the fourth stiffening cannula, in accordance with some embodiments.
FIG. 34 illustrates a third plurality of cutouts of the cannula tube of the fourth stiffening cannula, in accordance with some embodiments.
FIG. 35 illustrates the compound hub and the cannula tube of the fourth stiffening cannula, in accordance with some embodiments.
FIG. 36 illustrates the compound hub and the cannula tube of the fourth stiffening cannula, in accordance with some embodiments.
FIG. 37A illustrates a plunger in a well of a track in a dial of the compound hub of the fourth stiffening cannula, in accordance with some embodiments.
FIG. 37B illustrates a cross section of the compound hub of the fourth stiffening cannula with the plunger in a well of a track in the dial, in accordance with some embodiments.
FIG. 38A illustrates a plunger between wells of a track in the dial of the compound hub of the fourth stiffening cannula, in accordance with some embodiments.
FIG. 38B illustrates a cross section of the compound hub of the fourth stiffening cannula with the plunger between wells of a track in the dial, in accordance with some embodiments.
FIG. 39 illustrates a procedure involving placement of a TIPS between a hepatic vein and a portal vein, in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, portal hypertension is often treated by way of a percutaneous procedure involving placement of a TIPS between the hepatic vein and the portal vein as shown in FIG. 39 to establish blood flow through the liver. Placement of a portosystemic shunt between the right hepatic vein and the right portal vein is generally preferred, and transjugular liver access sets currently available are generally dedicated to this end. For example, bent or curved stiffening cannulas are often provided in the foregoing transjugular liver access sets to support an anterior needle throw from the right hepatic vein to the right portal vein. Being that the stiffening cannulas are already bent or curved, the stiffening cannulas do not easily accommodate anatomical variations in patients or different modes of access within the liver such as from the middle hepatic vein to either the left or right portal vein, which respectively requires either an anterior or posterior needle throw, or the left hepatic vein to the left portal vein, which requires a posterior needle throw. In view of the foregoing, the transjugular liver access sets currently available can inadvertently contribute to prolonged procedures and decreased success rates for patients having anatomical variations or needing different modes of access within the liver.

Disclosed herein are articulated stiffening cannulas, access sets, and methods thereof that address at least the forgoing shortcomings.

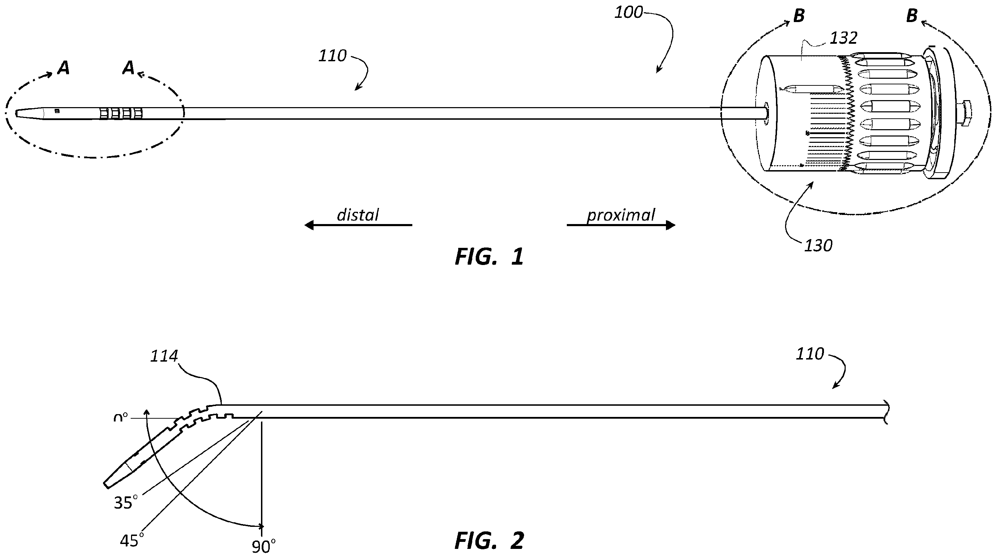

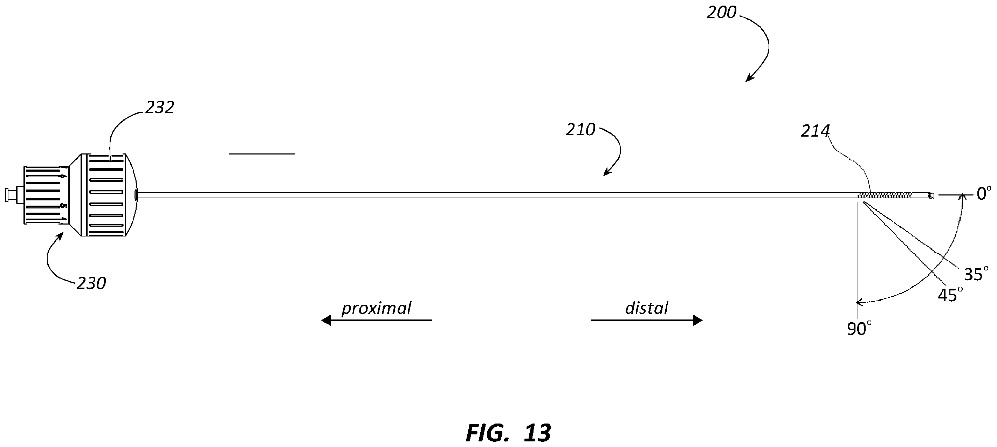

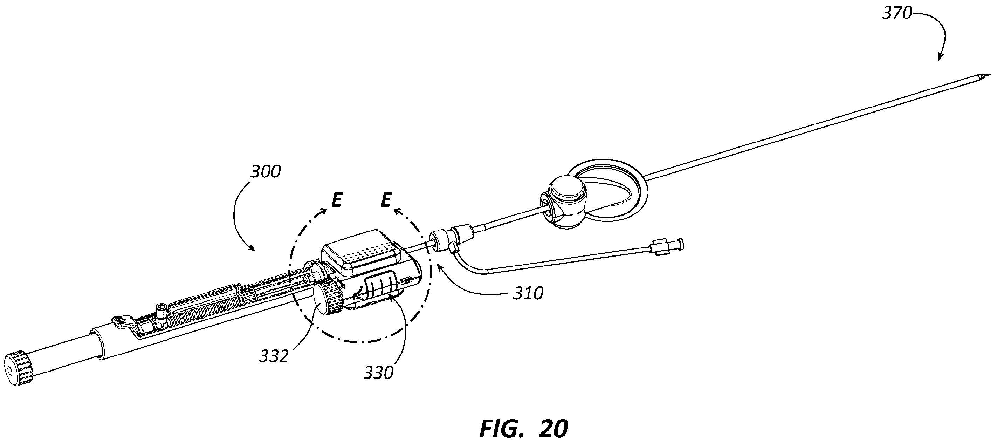

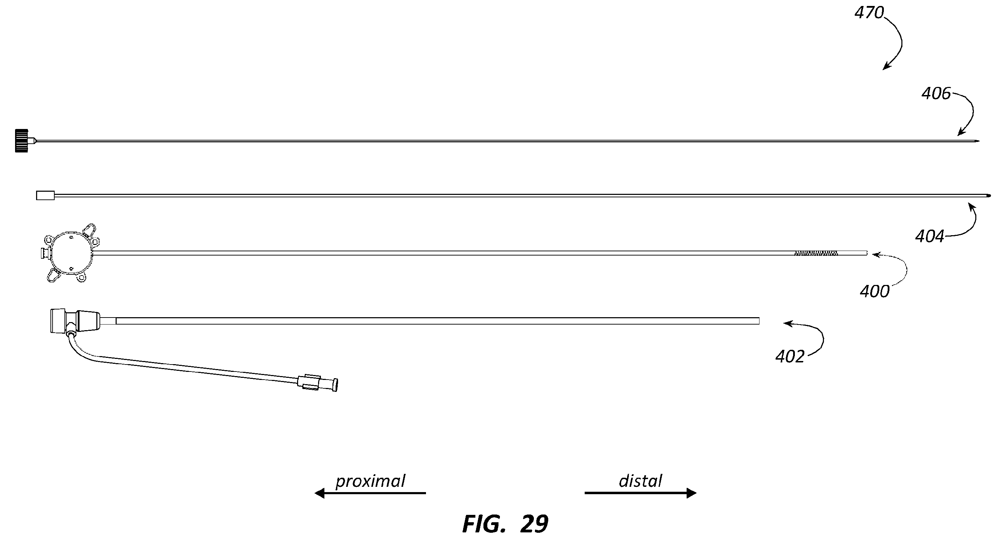

For example, an articulated stiffening cannula is disclosed such as the first stiffening cannula of FIGS. 1-12, the second stiffening cannula of FIGS. 13-19, the third stiffening cannula of FIGS. 20-28, and the fourth stiffening cannula of FIGS. 29-38. As set forth below, each stiffening cannula of the foregoing stiffening cannulas includes a cannula tube, a compound hub disposed about a proximal-end portion of the cannula tube, and either a stepwise or continuous-step articulation mechanism controlled by at least a rotatable element (e.g., rotatable hub, knob, dial, etc.) of the compound hub. The cannula tube includes a flexible hinge along a distal length of the cannula tube. The cannula tube is configured to articulate at the flexible hinge for adjusting a cannula-tube angle between a distal-end portion of the cannula tube and a proximal length of the cannula tube. The articulation mechanism includes either a single control line or a number of control lines extending from the compound hub, through the cannula tube beyond the flexible hinge, and to the distal-end portion of the cannula tube for adjusting the cannula-tube angle.

An articulated stiffening cannula of the first, second, third, or fourth stiffening cannulas supports anatomical variations in patients, different modes of access within the liver, or both the anatomical variations and the different modes of access. With respect to the support for the anatomical variations or the different modes of access, the cannula-tube angle of the stiffening cannula can be adjusted to any angle between about 0° and 180°, including about 0° and 90°, such as about 0° and 45°, for example, about 0° and 35° to accommodate the anatomical variations or the different modes of access. With further respect to the different modes of access, the stiffening cannula is configured to stiffen a catheter assembly such as a catheter-and-needle assembly in support of a needle throw from the catheter assembly at a chosen cannula-tube angle, which controls the direction of the needle throw. For example, the stiffening cannula stiffens the catheter assembly in a hepatic vein enabling an anterior needle throw from a right hepatic vein to a right portal vein, an anterior or posterior needle throw from a middle hepatic vein to a left portal vein or the right portal vein, or a posterior needle throw from a left hepatic vein to the left portal vein, in accordance with the chosen cannula-tube angle.

FIG. 1 illustrates a first articulated stiffening cannula 100, in accordance with some embodiments.

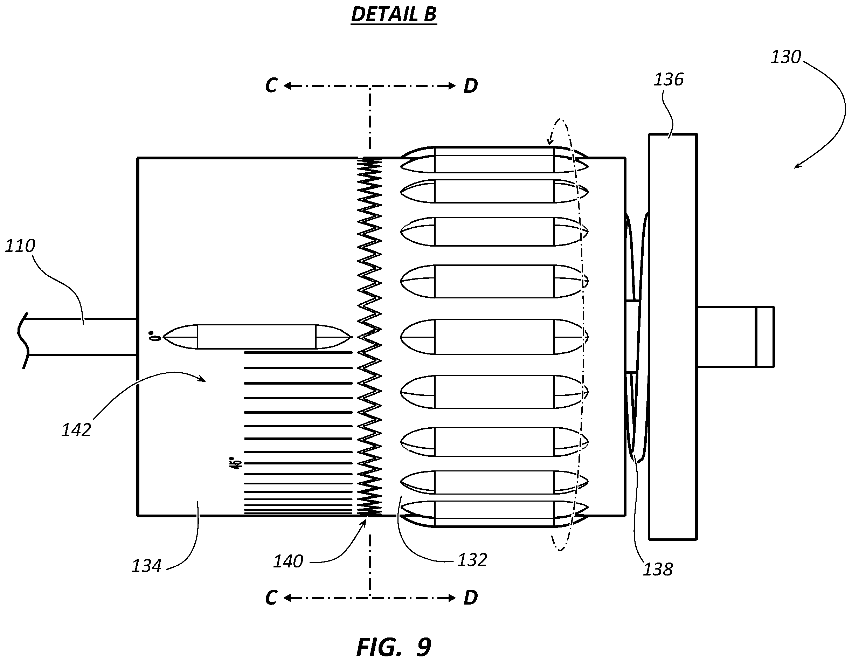

As shown, the stiffening cannula 100 includes a cannula tube 110, a compound hub 130 disposed about a proximal-end portion of the cannula tube, and a stepwise articulation mechanism controlled by at least a rotatable hub 132 (*see* FIG. 9) of the compound hub 130.

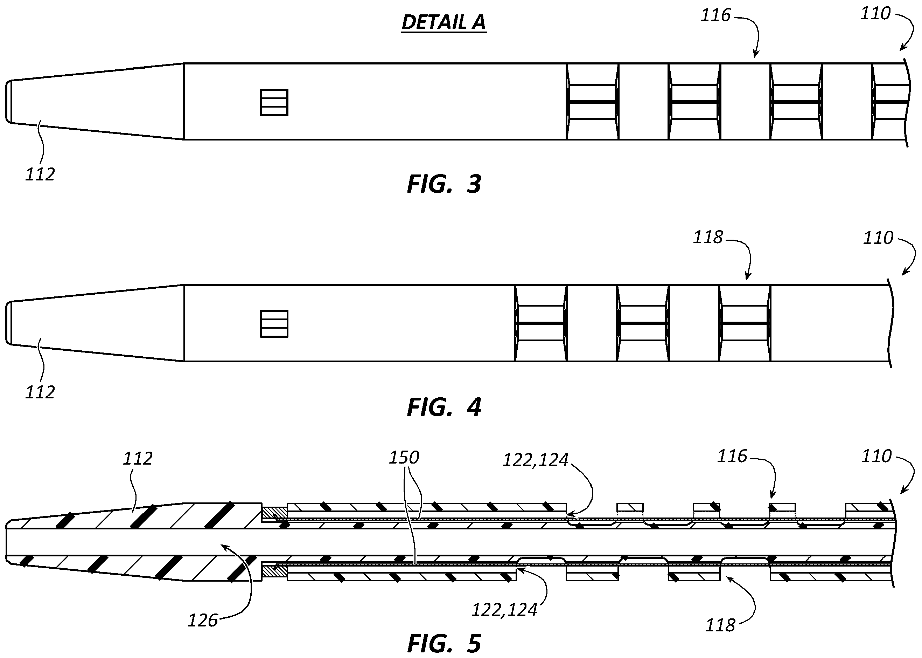

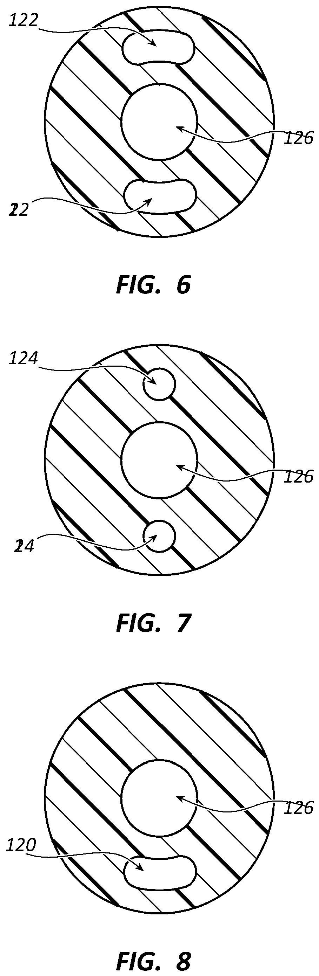

FIG. 2 illustrates the cannula tube 110 of the first stiffening cannula 100 adjusted in an arbitrary cannula-tube angle, in accordance with some embodiments. FIG. 3 illustrates a first set of a number of cutouts 116 along a first side of the cannula tube 110, in accordance with some embodiments. FIG. 4 illustrates a second set of the cutouts 118 along a second side of the cannula tube 110, in accordance with some embodiments. FIG. 5 illustrates a longitudinal cross section of the cannula tube 110, in accordance with some embodiments. FIGS. 6-8 illustrate transverse cross sections of the cannula tube 110, in accordance with some embodiments.

As shown, the cannula tube 110 includes a cone-shaped tip 112 and a flexible hinge 114 along a distal length of the cannula tube 110. The cannula tube 110 is configured to articulate at the flexible hinge 114 for adjusting the cannula-tube angle between a distal-end portion of the cannula tube 110 and a proximal length of the cannula tube 110. By way of example, the cannula-tube angle shown in FIG. 2 is about 35°; however, the cannula-tube angle can be adjusted to any angle between about 0° and 180°, including about 0° and 90°, such as about 0° and 45°, for example, about 0° and 35°.

The cannula tube 110 includes the cutouts, which are transverse cutouts including the first set of cutouts 116 along the first side of the cannula tube 110 and the second set of cutouts 118 along the second side of the cannula tube 110 opposite the first side of the cannula tube 110. The first set of cutouts 116 along the first side of the cannula tube 110 are greater in number of cutouts than the second set of the cutouts 118 along the first side of the cannula tube 110. The first set of cutouts 116 are also more narrowly spaced apart along the first side of the cannula tube 110 than the second set of the cutouts 118 along the first side of the cannula tube 110. Thus, the first set of cutouts 116 is configured for compression along the first side of the cannula tube 110 when the cannula tube 110 is articulated at the flexible hinge 114, while the second set of cutouts 118 is configured for tension along the second side of the cannula tube 110 when the cannula tube 110 is articulated at the flexible hinge 114. Alternatively, the first set of cutouts 116 is configured for tension along the first side of the cannula tube 110 when the cannula tube 110 is articulated at the flexible hinge 114, while the second set of cutouts 118 is configured for compression along the second side of the cannula tube 110 when the cannula tube 110 is articulated at the flexible hinge 114. Notwithstanding the foregoing, in some embodiments of the cannula tube 100 only one set of cutouts of the first set of cutouts 116 and the second set of cutout 118 are present. The first set of cutouts 116 and the second set of cutouts 118 can be laser cutouts, which are precise cutouts characterized by having edges with high-quality surface finishes. In addition, each cutout of the first set of cutouts 116 and the second set of cutouts 118 is filled with a pliable material including a polymeric material such as a polysiloxane, for example, a silicone. The flexible hinge 114 is formed at least in part by the pliable material-filled cutouts.

The cannula tube 110 includes a dedicated control-line lumen 120 extending through the cannula tube 110 alongside a needle lumen 126 of the cannula tube 110 or a pair of dedicated control-line lumens 122 or 124 extending through the cannula tube 110 alongside the needle lumen 126 such as on opposite sides of the needle lumen 126. The control-line lumen 120 and each control-line lumen of the pair of control-line lumens 122 is fabiform or reniform in transverse cross section while each control-line lumen of the pair of control-line lumens 124 is pisiform in transverse cross section; however, each control-line lumen of the control-line lumen 120 and the pairs of control-line lumens 122 or 124 can independently be fabiform, reniform, or pisiform in transverse cross section. A control line of the articulation mechanism is disposed in the control-line lumen 120, a pair of control lines 152 of the articulation mechanism is disposed in the control-line lumen 120, or each control line of the pair of control lines 152 is separately disposed in a control line lumen of the pair of control-line lumens 122 or 124. The needle lumen 126 is configured for disposing at least a 5-Fr catheter-and-needle assembly (e.g., a trocar needle disposed in a 5-Fr catheter) therein.

The cannula tube 110 terminates in a Luer-tapered fitting such as a slip or lock fitting at a proximal end of the cannula tube 110. The Luer-tapered fitting is convenient for connecting a syringe, which can be used to, for example, inject contrast medium for contrast-enhanced ultrasound to confirm a catheter is correctly placed across the liver parenchyma subsequent to a needle throw from the hepatic vein to the portal vein.

The cannula tube 110 is metal such as stainless steel or nitinol; however, the cannula tube 110 need not be limited to metal as the cannula tube 110 can alternatively be a composite such as any cannula tube of the cannula tubes 210, 310, 410.

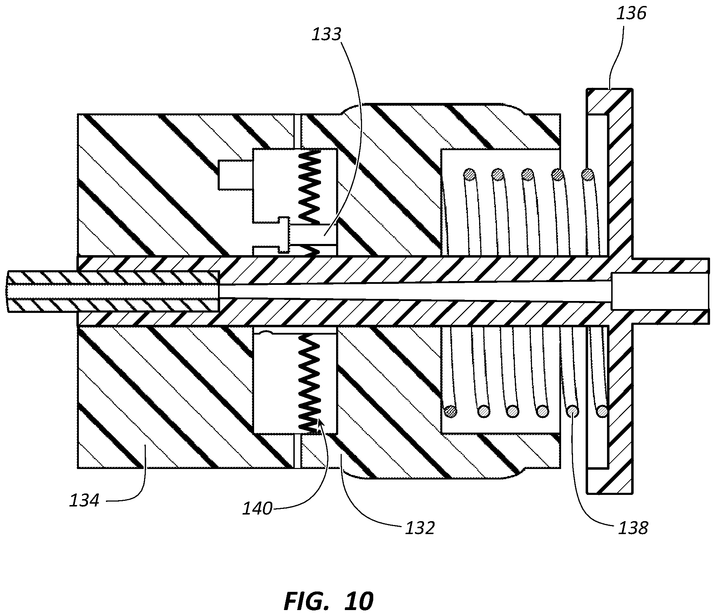

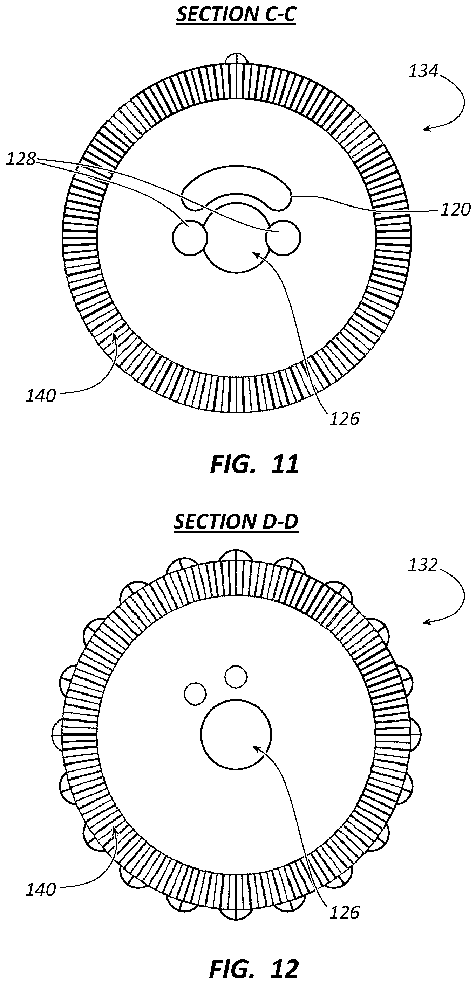

FIG. 9 illustrates the compound hub 130 of the first stiffening cannula 100, in accordance with some embodiments. FIG. 10 illustrates a longitudinal cross section of the compound hub 130, in accordance with some embodiments. FIGS. 11 and 12 illustrate transverse cross sections of the compound hub 130, in accordance with some embodiments.

As shown, the compound hub 130 includes the rotatable hub 132, a fixed hub 134, a fixed endpiece 136, and a compression spring 138. Both the fixed hub 134 and the fixed endpiece 136 are fixedly coupled to the cannula tube 110, whereas the rotatable hub 132 is rotatably coupled to the cannula tube 110, thereby configuring the rotatable hub 132 to rotate relative to the fixed hub 134 and the fixed endpiece 136. The rotatable hub 132 is disposed between the fixed hub 134 and the fixed endpiece 136 where the rotatable hub 132 is able to engage with the fixed hub 134 in a Hirth or curvic coupling 140. The rotatable hub 132 is held against the fixed hub 134 by a spring force of the compression spring 138, which is disposed between the rotatable hub 132 and the fixed endpiece 138, optionally in a bore of each of the rotatable hub 132 and the fixed endpiece 138. The Hirth or curvic coupling 140 is configured to hold the cannula tube 110 at the chosen cannula-tube angle in combination with the spring force of the compression spring 136.

The compound hub 130 optionally includes markings 142 on the rotatable hub 132, the fixed hub 134, or both the rotatable hub 132 and the fixed hub 134, the markings 142 including an indicator that can be followed when the cannula-tube angle is changed.

Each component of the compound hub 130 including the rotatable hub 132, the fixed hub 134, the fixed endpiece 136, and the compression spring 138 can be formed of a polymeric material such as polycarbonate or polypropylene; however, at least the compression spring 138 can alternatively be metal such as stainless steel.

The stepwise articulation mechanism includes a single control line or the pair of control lines 152 extending from the compound hub 130, through the cannula tube 110 beyond the flexible hinge 114, and to the distal-end portion of the cannula tube 110 for adjusting the cannula-tube angle. With respect to the pair of control lines 152, a proximal-end portion of a first control line of the pair of control lines 152 is coupled to the rotatable hub 132 through a direction-changing pillar 133 as shown in FIG. 10. A distal-end portion of the first control line is attached to the distal-end portion of the cannula tube 110 as shown in FIG. 5. Likewise, a proximal-end portion of a second control line of the pair of control lines 152 is coupled to the rotatable hub 132 through a corresponding direction-changing pillar. A distal-end portion of the second control line is attached to the distal-end portion of the cannula tube 110 as shown in FIG. 5.

The rotatable hub 132 is configured to draw the first control line into the compound hub 130 and let the second control line out of the compound hub 130 upon rotating the rotatable hub 132 in a first direction. Likewise, the rotatable hub 132 is configured to let the first control line out of the compound hub 130 and draw the second control line into the compound hub 130 upon rotating the rotatable hub 132 in a second direction. The first direction of the rotatable hub 132 increases the cannula-tube angle, and the second direction of the rotatable hub 132 decreases the cannula-tube angle.

FIG. 13 illustrates a second articulated stiffening cannula 200, in accordance with some embodiments.

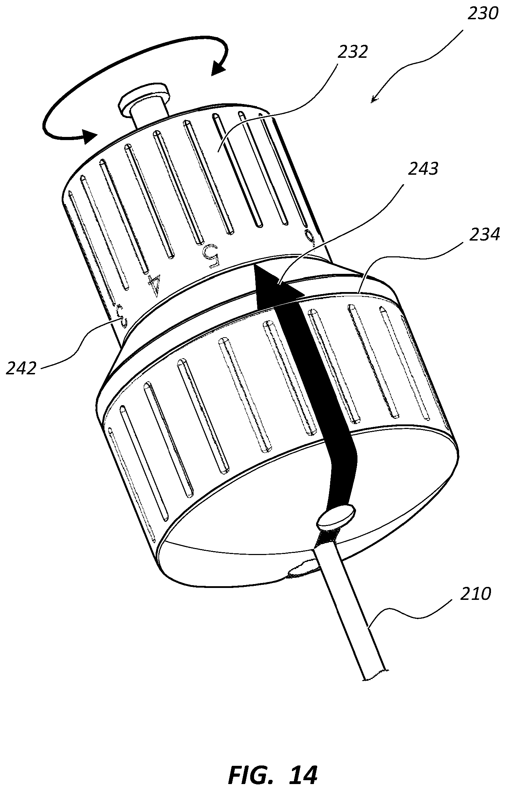

As shown, the stiffening cannula 200 includes a cannula tube 210, a compound hub 230 disposed about a proximal-end portion of the cannula tube 210, and a continuous-step articulation mechanism controlled by at least a rotatable hub 232 *(see* FIG. 14) of the compound hub 230.

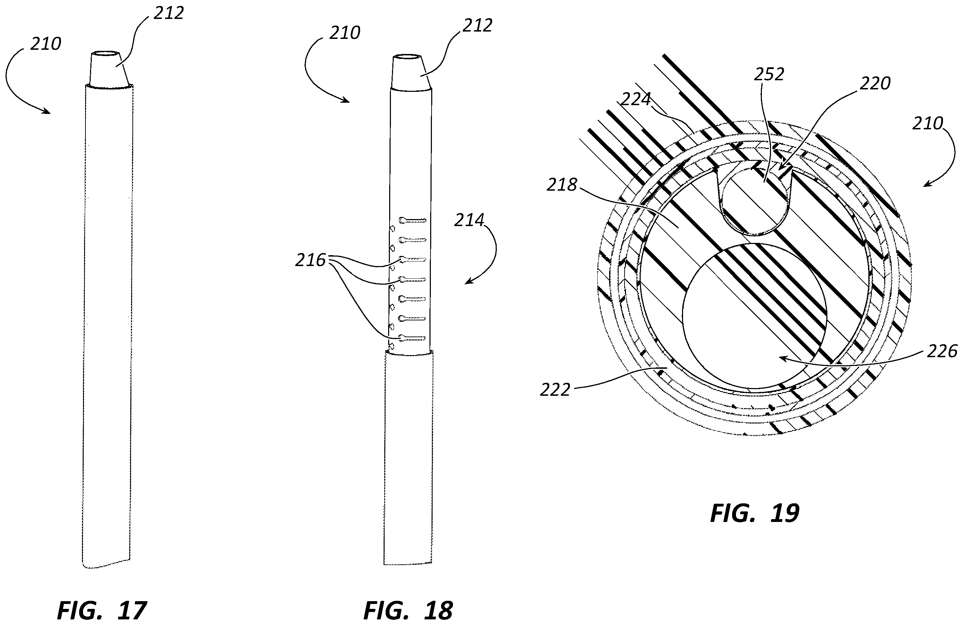

FIG. 17 illustrates a side of the cannula tube 210 of the second stiffening cannula 200 without cutouts or before cutting a number of cutouts in a side of the cannula tube 210, in accordance with some embodiments. FIG. 18 illustrates a number of cutouts 216 along the side of the cannula tube 210, in accordance with some embodiments. FIG. 19 illustrates a transverse cross section of the cannula tube 210, in accordance with some embodiments.

As shown, the cannula tube 210 includes an angled tip 212 configured for easy insertion into a body of a patient and a flexible hinge 214 along a distal length of the cannula tube 210. The cannula tube 210 is configured to articulate at the flexible hinge 214 for adjusting a cannula-tube angle between a distal-end portion of the cannula tube 210 and a proximal length of the cannula tube 210. *(See* FIG. 13.) By way of example, the cannula-tube angle shown in FIG. 13 is about 35°; however, the cannula-tube angle can be adjusted to any angle between about 0° and 180°, including about 0° and 90°, such as about 0° and 45°, for example, about 0° and 35°.

The cannula tube 210 includes the cutouts 216, which are transverse cutouts in one or more tubular layers along the side of the cannula tube 210. The cutouts 216 can be along a first side of the cannula tube 210, a second side of the cannula tube 210, or both the first side and the second side of the cannula tube 210. The cutouts 216 can be laser cutouts, which are precise cutouts characterized by having edges with high-quality surface finishes. The flexible hinge 214 is formed at least in part by the cutouts 216.

The cannula tube 210 includes a dedicated control-line lumen 220 extending through the cannula tube 210 alongside a needle lumen 226 of the cannula tube 210. The control-line lumen 220 is pisiform in transverse cross section; however, the control-line lumen 220 can be fabiform or reniform in transverse cross section. A single control line 252 of the articulation mechanism is disposed in the control-line lumen 220. The needle lumen 226 is configured for disposing at least a 5-Fr catheter-and-needle assembly (e.g., a trocar needle disposed in a 5-Fr catheter) therein.

The cannula tube 210 terminates in a Luer-tapered fitting such as a slip or lock fitting at a proximal end of the cannula tube 210. The Luer-tapered fitting is convenient for connecting a syringe, which can be used to, for example, inject contrast medium for contrast-enhanced ultrasound to confirm a catheter is correctly placed across the liver parenchyma subsequent to a needle throw from the hepatic vein to the portal vein.

The cannula tube 210 is a composite including a cannula-tube core 218 and one or more tubular layers disposed over the cannula-tube core 218 such as an inner layer 222 and an outer layer 224. Again, the cutouts 216 can be in one or more tubular layers along the side of the cannula tube 210 such as in the inner layer 222 and the outer layer 224 but not the cannula-tube core 218. The cannula-tube core 218 is metal such as stainless steel or nitinol, or the cannula-tube core 218 is a stiff polymeric material. Each layer of the one or more tubular layers is a polymeric material of a same or different polymeric material as an adjacent layer or the cannula-tube core 218 if the cannula-tube core 218 is also a polymeric material. The cannula tube 210 need not be limited to a composite as the cannula tube 210 can alternatively be metal such as the cannula tube 110.

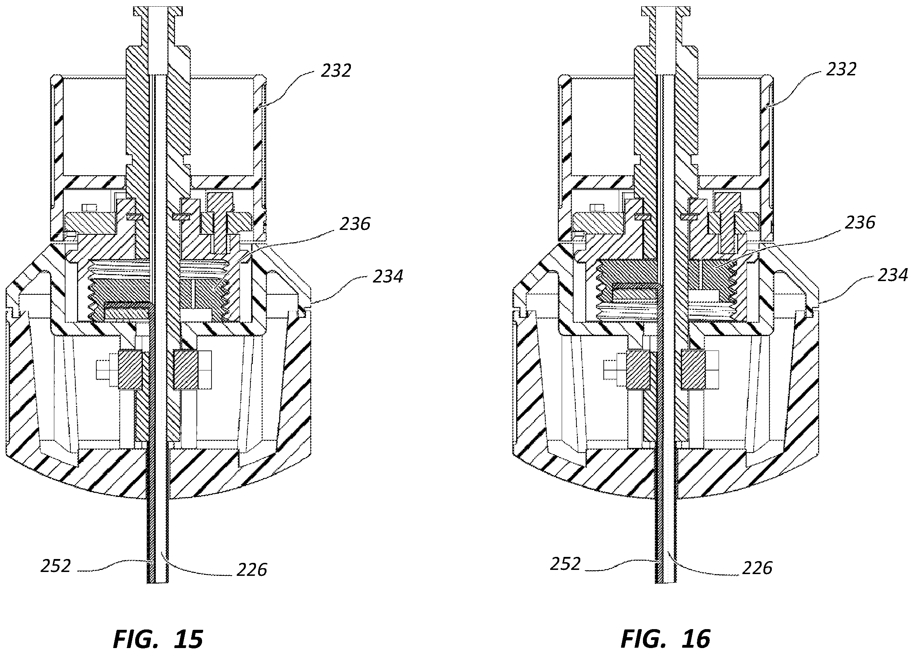

FIG. 14 illustrates a compound hub 230 of the second stiffening cannula 200, in accordance with some embodiments. FIG. 15 illustrates a longitudinal cross section of the compound hub 230 when the cannula-tube angle is about 0°, in accordance with some embodiments. FIG. 16 illustrates a longitudinal cross section of the compound hub 230 when the cannula-tube angle is at its greatest, in accordance with some embodiments.

As shown, the compound hub 230 includes the rotatable hub 232, a fixed hub 234, and a threaded plug 236. The fixed hub 234 is fixedly coupled to the cannula tube 210, whereas the rotatable hub 232 is rotatably coupled to the cannula tube 210, thereby configuring the rotatable hub 232 to rotate relative to the fixed hub 234. In addition, at least a portion of the rotatable hub 232, or an extension component thereof, is disposed in a bore in a proximal-end portion of the fixed hub 234. The rotatable hub 232 or the extension component thereof, in turn, includes a threaded bore concentric with the bore of the fixed hub 234. The threaded bore is configured to drive the threaded plug 236 proximally, away from the fixed hub 234 or distally, toward the fixed hub 234. Driving the threaded plug 236 away from the fixed hub 234 increases the cannula-tube angle, and driving the threaded plug 236 toward the fixed hub 234 decreases the cannula-tube angle.

The compound hub 230 optionally includes markings 242 on the rotatable hub 232, the fixed hub 234, or both the rotatable hub 232 and the fixed hub 234, the markings 242 including an indicator 243 that can be followed when the cannula-tube angle is changed.

Each component of the compound hub 230 including the rotatable hub 232, the fixed hub 234, and the threaded plug 236 can be formed of a polymeric material such as polycarbonate or polypropylene.

The continuous-step articulation mechanism includes the control line 252 extending from the compound hub 230, through the cannula tube 210 beyond the flexible hinge 214, and to the distal-end portion of the cannula tube 210 for adjusting the cannula-tube angle. A proximal-end portion of the control line 252 is coupled to the rotatable hub 232 through the threaded plug 236 as shown in FIGS. 15 and 16. A distal-end portion of the control line 252 is attached to the distal-end portion of the cannula tube 210 akin to how the first or second control lines of the pair of control lines 152 are attached to the distal-end portion of the cannula tube 110 as shown in FIG. 5.

The rotatable hub 232 is configured to draw the control line 252 into the compound hub 230 upon rotating the rotatable hub 232 in a first direction. The rotatable hub 232 is also configured to let the control line 252 out of the compound hub 230 upon rotating the rotatable hub 232 in a second direction opposite the first direction. The first direction of the rotatable hub 232 drives the threaded plug 236 away from the fixed hub 234, thereby increasing the cannula-tube angle. The second direction of the rotatable hub 232 drives the threaded plug 236 toward the fixed hub 234, thereby decreasing the cannula-tube angle.

FIG. 20 illustrates a first access set 370 including a third articulated stiffening cannula 300, in accordance with some embodiments.

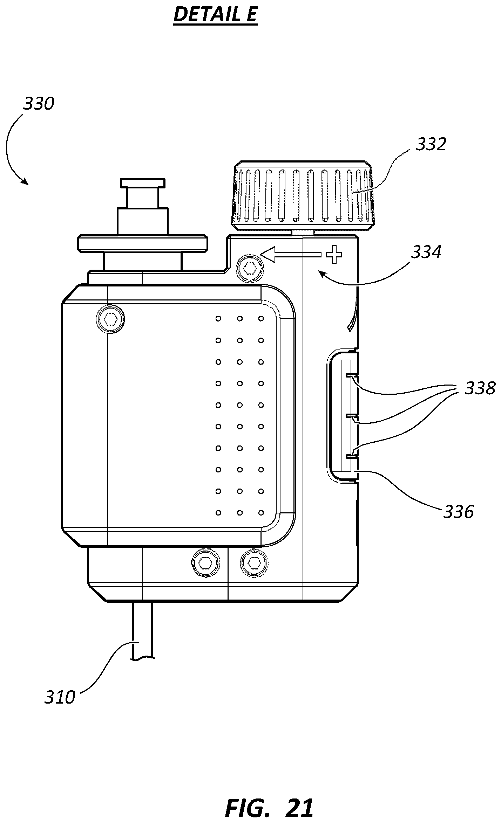

As shown, the stiffening cannula 300 includes a cannula tube 310, a compound hub 330 having a body disposed about a proximal-end portion of the cannula tube 310, and a continuous-step articulation mechanism controlled by at least a rotatable knob 332 *(see* FIG. 21) of the compound hub 330.

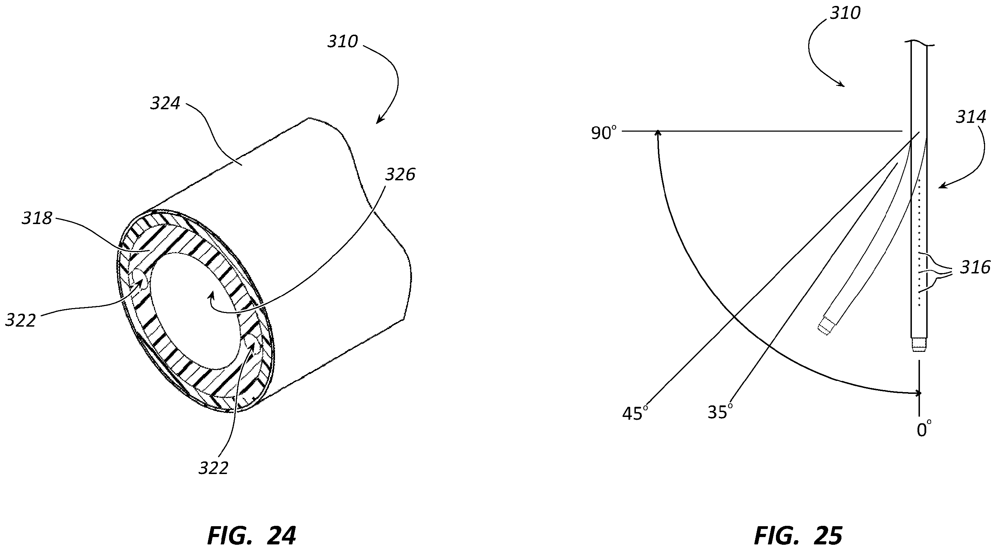

FIG. 24 illustrates a transverse cross section of the cannula tube 310 of the third stiffening cannula 300, in accordance with some embodiments. FIG. 25 illustrates the cannula tube 310 adjusted in an arbitrary cannula-tube angle, in accordance with some embodiments.

As shown, the cannula tube 310 includes a flexible hinge 314 along a distal length of the cannula tube 310. The cannula tube 310 is configured to articulate at the flexible hinge 314 for adjusting a cannula-tube angle between a distal-end portion of the cannula tube 310 and a proximal length of the cannula tube 310. By way of example, the cannula-tube angle shown in FIG. 25 is about 35°; however, the cannula-tube angle can be adjusted to any angle between about 0° and 180°, including about 0° and 90°, such as about 0° and 45°, for example, about 0° and 35°.

The cannula tube 310 includes a number of cutouts 316, which are transverse cutouts in one or more tubular layers along the side of the cannula tube 310. The cutouts 316 can be along a first side of the cannula tube 310, a second side of the cannula tube 310, or both the first side and the second side of the cannula tube 310. The cutouts 316 can be laser cutouts, which are precise cutouts characterized by having edges with high-quality surface finishes. The flexible hinge 314 is formed at least in part by the cutouts 316.

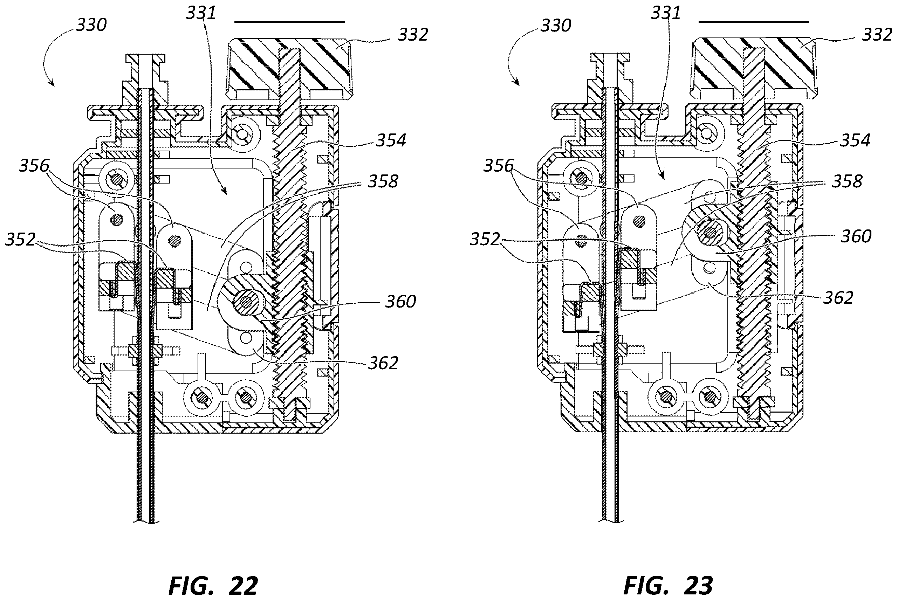

The cannula tube 310 includes a pair of dedicated control-line lumens 322 extending through the cannula tube 310 alongside the needle lumen 326 such as on opposite sides of the needle lumen 326. Each control-line lumen of the pair of control-line lumens 322 is pisiform in transverse cross section; however, each control-line lumen of the pair of control-line lumens 322 can independently be fabiform, reniform, or pisiform in transverse cross section. Each control line of a pair of control lines 352 *(see* FIGS. 22 and 23) is separately disposed in a control line lumen of the pair of control-line lumens 322. The needle lumen 326 is configured for disposing at least a 5-Fr catheter-and-needle assembly (e.g., a trocar needle disposed in a 5-Fr catheter) therein.

The cannula tube 310 terminates in a Luer-tapered fitting such as a slip or lock fitting at a proximal end of the cannula tube 310. *(See* FIGS. 22 and 23.) The Luer-tapered fitting is convenient for connecting a syringe, which can be used to, for example, inject contrast medium for contrast-enhanced ultrasound to confirm a catheter is correctly placed across the liver parenchyma subsequent to a needle throw from the hepatic vein to the portal vein.

The cannula tube 310 is a composite including a cannula-tube core 318 and one or more tubular layers disposed over the cannula-tube core 318 such as an outer layer 324. Again, the cutouts 316 can be in one or more tubular layers along the side of the cannula tube 310 such as in the outer layer 324 but not the cannula-tube core 318. The cannula-tube core 318 is metal such as stainless steel or nitinol, or the cannula-tube core 318 is a stiff polymeric material. Each layer of the one or more tubular layers is a polymeric material of a same or different polymeric material as an adjacent layer or the cannula-tube core 318 if the cannula-tube core 318 is also a polymeric material. The cannula tube 310 need not be limited to a composite as the cannula tube 310 can alternatively be metal such as the cannula tube 110.

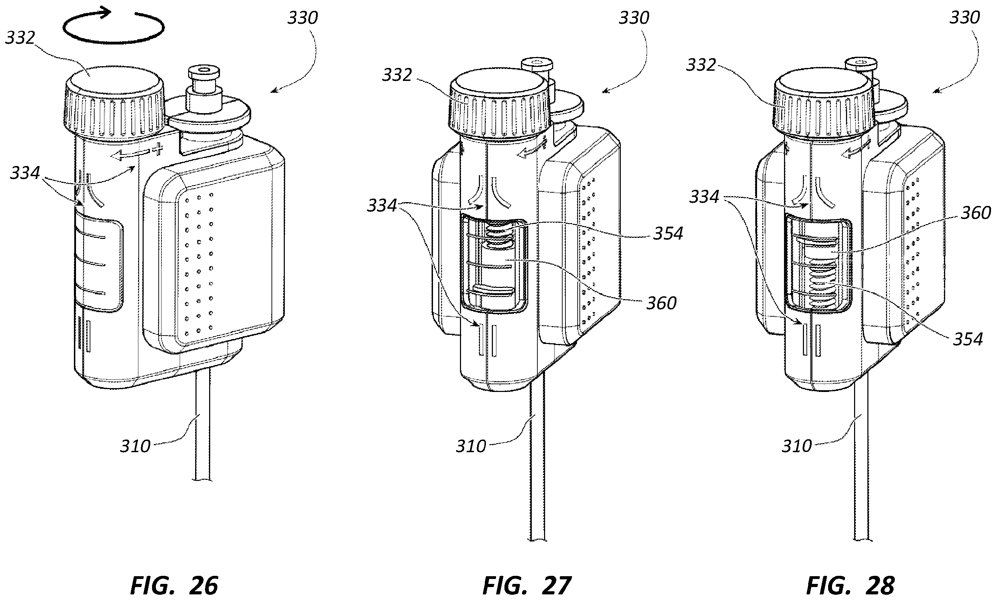

FIG. 21 illustrates the compound hub 330 of the third stiffening cannula 300, in accordance with some embodiments. FIG. 22 illustrates a longitudinal cross section of the compound hub 330 when the cannula-tube angle is about 0°, in accordance with some embodiments. FIG. 23 illustrates a longitudinal cross section of the compound hub 330 when the cannula-tube angle is at its greatest, in accordance with some embodiments. FIG. 26 illustrates markings on the compound hub 330 for increasing the cannula-tube angle, in accordance with some embodiments. FIG. 27 illustrates a window of the compound hub 330 having cannula-tube-angle graduations, in accordance with some embodiments. FIG. 28 illustrates the window of the compound hub 330 having the cannula-tube-angle graduations, in accordance with some embodiments.

As shown, the compound hub 330 includes the rotatable knob 332 fixedly coupled to a leadscrew 354 of the articulation mechanism, thereby configuring the rotatable knob 332 to rotate relative to the body of the compound hub 330.

The compound hub 330 includes markings 334 molded or printed on the body of the compound hub 330. The markings 334 include those indicating a first direction for rotating the knob 332 to increase the cannula-tube angle, a second direction for rotating the knob 332 to decrease the cannula-tube angle.

The compound hub 330 includes a window 336 disposed in a window opening of the body of the compound hub 330. The window 336 includes graduations 338 molded or printed on the window 336 for following an indictor (e.g., an arrow, a line, etc.) molded or printed on a window-facing side of a leadscrew-nut block 360 of the articulation mechanism. The leadscrew-nut is behind the graduations 338 of the window 336 such that the indicator can be followed when the cannula-tube angle is changed. In addition, the markings 334 include those on the body of the compound hub 330 adjacent ends of the window 336, which markings pictorially show extremes of the cannula-tube angle for use with the indicator and the graduations 338.

Each component of the compound hub 330 including the rotatable knob 332 and the body of the compound hub 330 can be formed of a polymeric material such as polycarbonate or polypropylene.

The continuous-step articulation mechanism includes the pair of control lines 352 tensioned and extending from the compound hub 330, through the cannula tube 310 beyond the flexible hinge 314, and to the distal-end portion of the cannula tube 310 for adjusting the cannula-tube angle. A proximal-end portion of a first control line of the pair of control lines 352 is mounted on a first control-line mount of a pair of control-line mounts 356 as shown in FIGS. 22 and 23. A proximal-end portion of a second control line of the pair of control lines 352 is mounted on a second control-line mount of the pair of control-line mounts 356. A distal-end portion of each of the first control line and the second control line is attached to a location opposite the other at the distal-end portion of the cannula tube 310 akin to how the first or second control lines of the pair of control lines 152 are attached to the distal-end portion of the cannula tube 110 as shown in FIG. 5. Each mount of the first control-line mount and the second control-line mount is rotatably coupled to each control arm a pair of control arms 358. Each control arm of the pair of control arms 358 is, in turn, rotatably coupled to a follower 362 of the leadscrew-nut block 360. The leadscrew-nut block 360 is fastened to the leadscrew 354 by way of complementary threads of each of the leadscrew-nut block 360 and the leadscrew 354.

The rotatable knob 332 is configured to draw the first control line into the compound hub 330 and let the second control line out of the compound hub 330 upon rotating the rotatable knob 332 in a first direction. Likewise, the rotatable knob 332 is configured to let the first control line out of the compound hub 330 and draw the second control line into the compound hub 330 upon rotating the rotatable knob 332 in a second direction opposite the first direction. The first direction of the rotatable knob 332 increases the cannula-tube angle, and the second direction of the rotatable knob decreases the cannula-tube angle.

The rotatable knob 332 is rotated as far as possible in the second direction in FIG. 22, thereby providing the cannula-tube angle at about 0°. This is shown by way of the leadscrew-nut block 360 and the follower 362 bottoming out in a cavity 331 of the body of the compound hub 330 in which the leadscrew-nut block 360 and the follower 362 are disposed. The rotatable knob 332 is rotated as far as possible in the first direction in FIG. 23, thereby providing the cannula-tube angle at its greatest extent. This is shown by way of the leadscrew-nut block 360 and the follower 362 topping out in the cavity 331 of the compound hub 330 in which the leadscrew-nut block 360 and the follower 362 are disposed.

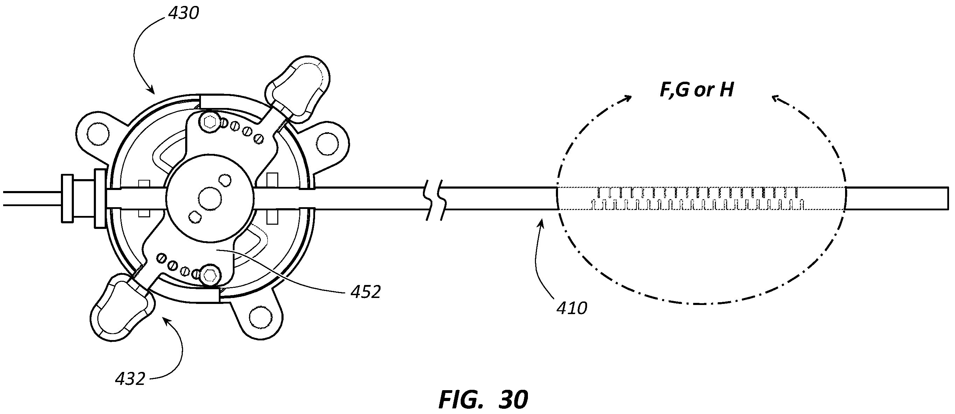

FIG. 29 illustrates a second access set 470 including a fourth articulated stiffening cannula 400, in accordance with some embodiments. FIG. 30 illustrates the fourth stiffening cannula 400, in accordance with some embodiments.

As shown, the second access set 470 includes an introducer sheath 402, the stiffening cannula 400, a catheter 404, and a needle 406.

The stiffening cannula 400 includes a cannula tube 410, a compound hub 430 having a body disposed about a proximal-end portion of the cannula tube 410, and a stepwise articulation mechanism controlled by a dial 452 (*see* FIG. 30) of the compound hub 430.

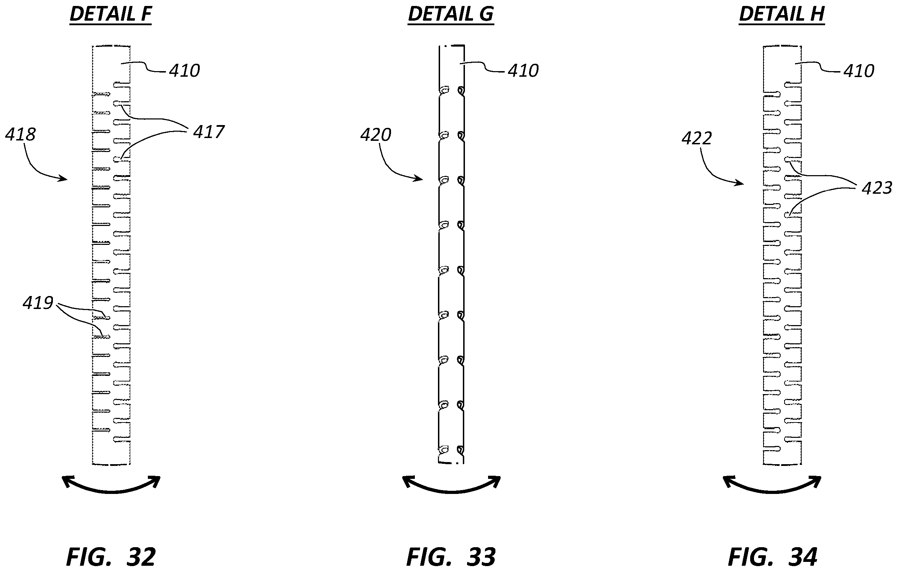

FIG. 32 illustrates a first plurality of cutouts 418 of the cannula tube 410 of the fourth stiffening cannula, in accordance with some embodiments. FIG. 33 illustrates a second plurality of cutouts 420 of the cannula tube 410, in accordance with some embodiments. FIG. 34 illustrates a third plurality of cutouts 422 of the cannula tube 410, in accordance with some embodiments.

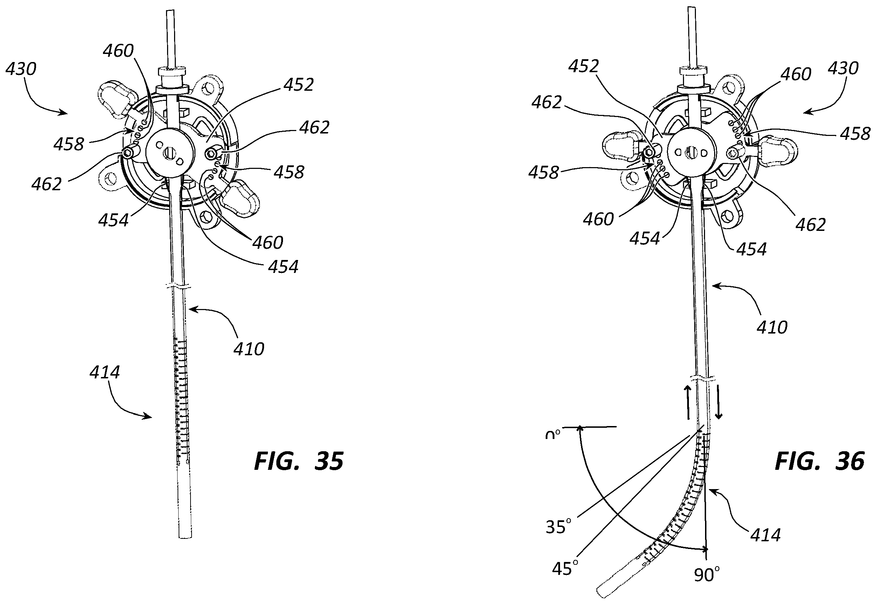

As shown, the cannula tube 410 includes a flexible hinge 414 along a distal length of the cannula tube 410. The cannula tube 410 is configured to articulate at the flexible hinge 414 for adjusting a cannula-tube angle between a distal-end portion of the cannula tube 410 and a proximal length of the cannula tube 410. (*See* FIG. 36.) By way of example, the cannula-tube angle shown in FIG. 36 is about 35°; however, the cannula-tube angle can be adjusted to any angle between about 0° and 180°, including about 0° and 90°, such as about 0° and 45°, for example, about 0° and 35°.

The cannula tube 410 includes the first plurality of cutouts 418, the second plurality of cutouts 420, or the third plurality of cutouts 422, the cutouts of which are transverse cutouts in one or more tubular layers along opposing sides of the cannula tube 410. The cutouts 418, 420, or 422 can be laser cutouts, which are precise cutouts characterized by having edges with high-quality surface finishes. The flexible hinge 414 is formed at least in part by the cutouts 418, 420, or 422.

The first plurality of cutouts 418 includes a first set of cutouts 417 along a first side of the cannula tube 410 and a second set of cutouts 419 along a second side of the cannula tube 410 opposite the first side of the cannula. The first set of cutouts 417 along the first side of the cannula tube 410 is staggered with the second set of cutouts 419 along the second side of the cannula tube 410; however, the first set of cutouts 417 need not be staggered with the second set of cutouts 419 in other embodiments. When viewed from a third or fourth side of the cannula tube 410 as in FIG. 32, each cutout of the cutouts 418 is a slot in the cannula tube 410, wherein each cutout of the first set of cutouts 417 is wider than a cutout of the second set of cutouts 419. With relatively less material of the cannula tube 410 between the cutouts of the first set of cutouts 417 than the cutouts of the second set of cutouts 419, the first set of cutouts 417 is more aptly configured for compression when the cannula tube 410 is articulated at the flexible hinge 414. Thus, the second set of cutouts 419 is configured for tension when the cannula tube 410 is articulated at the flexible hinge 414.

The second plurality of cutouts 420 includes cutouts along the first side of the cannula tube 410 and cutouts along the second side of the cannula tube 410 opposite the first side of the cannula. The cutouts along the first side of the cannula tube 410 are aligned with the cutouts 419 along the second side of the cannula tube 410; however, the cutouts along the first side of the cannula tube 410 need not be aligned with the cutouts along the second side of the cannula tube 410 in other embodiments. When viewed from a third or fourth side of the cannula tube 410 as in FIG. 33, each cutout of the cutouts 420 is a slot in the cannula tube 410 of approximately equal width to each cutout of the other cutouts 420. Thus, the cutouts along the first side of the cannula tube 410 and the cutouts along the second side of the cannula tube 410 are configured for either compression or tension when the cannula tube 410 is articulated at the flexible hinge 414.

The third plurality of cutouts 422 includes cutouts along the first side of the cannula tube 410 and cutouts along the second side of the cannula tube 410 opposite the first side of the cannula. The cutouts along the first side of the cannula tube 410 are staggered with the cutouts 419 along the second side of the cannula tube 410; however, the cutouts along the first side of the cannula tube 410 need not be staggered with the cutouts along the second side of the cannula tube 410 in other embodiments. When viewed from a third or fourth side of the cannula tube 410 as in FIG. 34, each cutout of the cutouts 422 is a clithridiate slot in the cannula tube 410 of approximately equal dimensions to each cutout of the other cutouts 422. Thus, the cutouts along the first side of the cannula tube 410 and the cutouts along the second side of the cannula tube 410 are configured for either compression or tension when the cannula tube 410 is articulated at the flexible hinge 414.

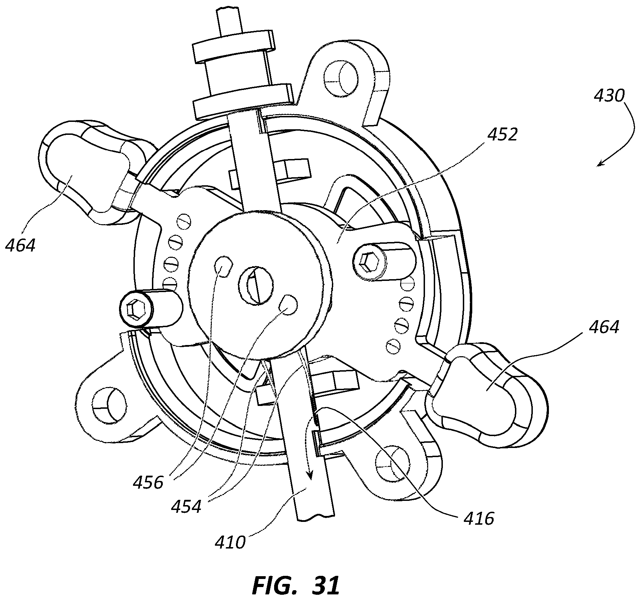

The cannula tube 410 includes a pair of dedicated control-line lumens 416 extending through the cannula tube 410 alongside a needle lumen (not shown) such as on opposite sides of the needle lumen. Each control-line lumen of the pair of control-line lumens 416 is pisiform in transverse cross section; however, each control-line lumen of the pair of control-line lumens 416 can independently be fabiform, reniform, or pisiform in transverse cross section. Each control line of a pair of control lines 454 (*see* FIG. 31) is separately disposed in a control line lumen of the pair of control-line lumens 416. The needle lumen is configured for disposing at least a 5-Fr catheter-and-needle assembly (e.g., a trocar needle disposed in a 5-Fr catheter) therein.

The cannula tube 410 terminates in a Luer-tapered fitting such as a slip or lock fitting at a proximal end of the cannula tube 410. (*See* FIG. 30.) The Luer-tapered fitting is convenient for connecting a syringe, which can be used to, for example, inject contrast medium for contrast-enhanced ultrasound to confirm a catheter is correctly placed across the liver parenchyma subsequent to a needle throw from the hepatic vein to the portal vein.

The cannula tube 410 is a composite including a cannula-tube core and one or more tubular layers disposed over the cannula-tube core. Again, the cutouts 418, 420, or 422 can be in one or more tubular layers along the sides of the cannula tube 410 such as in an outer layer but not the cannula-tube core. The cannula-tube core is metal such as stainless steel or nitinol, or the cannula-tube core is a stiff polymeric material. Each layer of the one or more tubular layers is a polymeric material of a same or different polymeric material as an adjacent layer or the cannula-tube core if the cannula-tube core is also a polymeric material. The cannula tube 410 need not be limited to a composite as the cannula tube 410 can alternatively be metal such as the cannula tube 110.

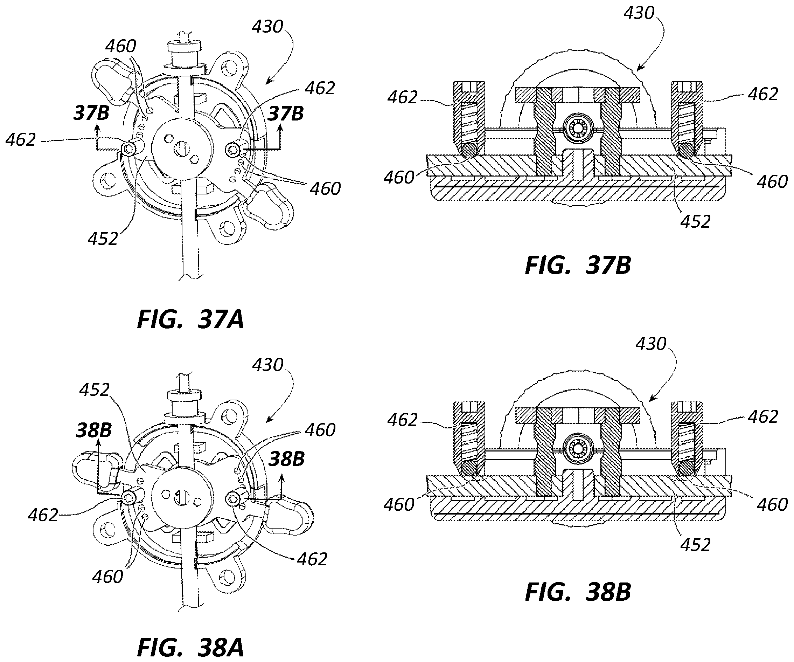

FIG. 31 illustrates the compound hub 430 of the fourth stiffening cannula 400, in accordance with some embodiments. FIG. 35 illustrates the compound hub 430 and the cannula tube 410, in accordance with some embodiments. FIG. 36 illustrates the compound hub 430 and the cannula tube 410, in accordance with some embodiments. FIGS. 37A and 37B illustrate different views of a plunger 462 in a well of a track in a dial 452 of the compound hub 430, in accordance with some embodiments. FIGS. 38A and 38B illustrate different views of a plunger 462 between wells of a track in the dial 452 of the compound hub 430, in accordance with some embodiments.

While not shown, the compound hub 430 includes a cover either removably or fixedly coupled to the body of the compound hub, thereby forming an enclosure for the articulation mechanism.

Each component of the compound hub 430 including the body and the cover of the compound hub 430 can be formed of a polymeric material such as polycarbonate or polypropylene.

The stepwise articulation mechanism includes the pair of control lines 454 tensioned and extending from the compound hub 430, through the cannula tube 410 beyond the flexible hinge 414, and to the distal-end portion of the cannula tube 410 for adjusting the cannula-tube angle. A proximal-end portion of a first control line of the pair of control lines 454 is attached to a first control-line pin of a pair of control-line pins 456 disposed in an inner annulus of the dial 452 as shown in FIGS. 31, 35 and 36. A proximal-end portion of a second control line of the pair of control lines 454 is attached to a second control-line pin of control-line pins 456 disposed in the inner annulus of the dial 452 as shown in FIGS. 31, 35 and 36. The cannula tube 410 is between the first control-line pin and the second control-line pin. A distal-end portion of each of the first control line and the second control line is attached to a location opposite the other at the distal-end portion of the cannula tube 410 akin to how the first or second control lines of the pair of control lines 152 are attached to the distal-end portion of the cannula tube 110 as shown in FIG. 5.

A pair of opposing extension tabs 464 is coupled to the dial 452. Each extension tab of the pair of extension tabs 464 extends from an arcuate slot in the body of the compound hub 430 for controlling the dial 452. Each pin of the pair of control-line pins 456 is configured to subtend a same angle as a corresponding extension tab of the extension tabs 464 when the dial 452 is rotated. The corresponding extension tab of the first control-line pin or the second control-line pin tab is on a same side of the cannula tube 410 as the first control-line pin or the second control-line pin.

The dial 452 includes a pair of arcuate tracks 458 in the dial 452. A first track of the pair of tracks 458 is disposed in an outer annulus of the dial 452. A second track of the pair of tracks 458 is disposed in the outer annulus of the dial 452. The cannula tube 410 is between the first track and the second track. Each track of the first track and the second track includes a number of wells 460. Each well of the wells 460 in the first track or the second track is a predetermined arcuate distance from another well in the first track or the second track. The arcuate distance corresponds to a stepwise change in the cannula-tube angle.

A spring-loaded plunger 462 of the first track or the second track of the pair of tracks 458 is on a same side of the cannula tube 410 as the first track or the second track. The plunger 462 of the first track or the second track is configured to insert into the wells 460 of the first track or the second track. In other words, the wells 460 of each track of the first track and the second track are configured to accept the corresponding spring-loaded plunger 462 disposed therein. The plunger 462 corresponding to the first track or the second track makes audible clicks when the dial 452 is rotated. Each audible click of the audible clicks corresponds to disposal of the plunger 452 in a well of the wells 460.

The dial 452 is configured to draw the first control line into the compound hub 430 and let the second control line out of the compound hub upon rotating the dial in a first direction. Likewise, the dial 452 is configured to let the first control line out of the compound hub and draw the second control line into the compound hub upon rotating the dial in a second direction opposite the first direction. The first direction of the dial 452 increases the cannula-tube angle, and the second direction of the dial 452 decreases the cannula-tube angle.

The dial 452 is rotated as far as possible in the second direction in FIG. 35, thereby providing the cannula-tube angle at about 0°. This is shown by way of the plunger 452 at a first end of each track of the first track or the second track. The dial 452 is rotated as far as possible in the first direction in FIG. 36, thereby providing the cannula-tube angle at its greatest extent. This is shown by way of the plunger 452 at second end of each track of the first track or the second track opposite the first end.

### Access sets

An access set includes an introducer sheath, an articulated stiffening cannula, a catheter, and a needle (e.g., trocar needle). For example, the access set can be the access set 470 including the introducer sheath 402, the catheter 404, the needle 406, and any stiffening cannula of the stiffening cannulas 100, 200, 300, and 400. The stiffening cannula is configured to stiffen at least a catheter-and-needle assembly in support of a needle throw at a chosen cannula-tube angle from the catheter-and-needle assembly when disposed in the stiffening cannula. The catheter-and-needle assembly includes the needle disposed in the catheter providing, for example, at least a 5-Fr catheter-and-needle assembly. The cannula tube of the stiffening cannula includes a needle lumen longitudinally extending through the cannula tube configured for disposing the catheter-and-needle assembly therein.

### Methods

A method of an articulated stiffening cannula includes inserting a stiffening cannula of the stiffening cannulas 100, 200, 300, and 400 into an introducer sheath such as the introducer sheath 402 positioned in a distal portion of a hepatic vein, the stiffening cannula including a cannula tube having a flexible hinge along a distal length of the cannula tube for adjusting a cannula-tube angle between a distal-end portion of the cannula tube and a proximal length of the cannula tube; inserting a catheter-and-needle assembly into the stiffening cannula, the catheter-and-needle assembly including, for example, the needle 406 disposed in the catheter 404, thereby stiffening at least the catheter-and-needle assembly; articulating the stiffening cannula by rotating a rotatable element of a compound hub of the stiffening cannula either before or after inserting the catheter-and-needle assembly; choosing the cannula-tube angle for a needle throw from the hepatic vein to a portal vein; and throwing the needle from the hepatic vein to the portal vein.

Throwing the needle from the hepatic vein to the portal vein includes throwing the needle from a right hepatic vein to a right portal vein with an anterior needle throw in accordance with the cannula-tube angle chosen for the needle throw. Alternatively, throwing the needle from the hepatic vein to the portal vein includes throwing the needle from a middle hepatic vein to either a left portal vein or a right portal vein respectively with either an anterior needle throw or a posterior needle throw in accordance with the cannula-tube angle chosen for the needle throw. Alternatively, throwing the needle from the hepatic vein to the portal vein includes throwing the needle from a left hepatic vein to a left portal vein with a posterior needle throw in accordance with the cannula-tube angle chosen for the needle throw.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

The invention is defined by the following claims.

## Claims

1. An articulated stiffening cannula (100, 200, 300, 400), comprising:
a cannula tube (110, 210, 310, 410) including a flexible hinge (114, 214, 314, 414) along a distal length of the cannula tube (110, 210, 310, 410), the cannula tube (110, 210, 310, 410) configured to articulate at the flexible hinge (114, 214, 314, 414) for adjusting a cannula-tube angle between a distal-end portion of the cannula tube (110, 210, 310, 410) and a proximal length of the cannula tube (110, 210, 310, 410);
a compound hub (130, 230, 330, 430) disposed about a proximal-end portion of the cannula tube (110, 210, 310, 410), wherein the compound hub (130, 230, 330, 430) includes a fixed hub (134, 234) fixedly coupled to the cannula tube (110, 210, 310, 410) and a rotatable hub (132, 232) configured to rotate relative to the fixed hub (134, 234); and
an articulation mechanism controlled by at least a rotatable element of the compound hub (130, 230, 330, 430), the articulation mechanism including a single control line (152, 252, 352, 454) or a pair of control lines (152, 252, 352, 454) extending from the compound hub (130, 230, 330, 430), through the cannula tube (110, 210, 310, 410) beyond the flexible hinge (114, 214, 314, 414), and to the distal-end portion of the cannula tube (110, 210, 310, 410) for adjusting the cannula-tube angle, the stiffening cannula (100, 200, 300, 400) configured to stiffen a catheter assembly in support of a needle throw from the catheter assembly at a chosen cannula-tube angle,
wherein the articulation mechanism is controlled by at least the rotatable hub (132, 232)
wherein the single control line (152, 252, 352, 454) or each control line (152, 252, 352, 454) of the pair of control lines (152, 252, 352, 454) is disposed in a separate, dedicated control-line lumen (120, 122, 124, 220, 322, 416) longitudinally extending through the cannula tube (110, 210, 310, 410) alongside a needle lumen (126, 226, 326) of the cannula tube (110, 210, 310, 410), or
wherein both the control lines (152, 252, 352, 454) of the pair of control lines (152, 252, 352, 454) are disposed in a dedicated control-line lumen (120, 122, 124, 220, 322, 416) extending through the cannula tube (110, 210, 310, 410) alongside a needle lumen (126, 226, 326) of the cannula tube (110, 210, 310, 410).

2. The articulated stiffening cannula (100, 200, 300, 400) of claim 1, wherein the cannula tube (110, 210, 310, 410) includes a plurality of transverse cutouts (116, 118, 216, 316, 417, 418, 419, 420, 422) in the cannula tube (110, 210, 310, 410), the flexible hinge (114, 214, 314, 414) formed at least in part by the cutouts (116, 118, 216, 316, 417, 418, 419, 420, 422).

3. The articulated stiffening cannula (100, 200, 300, 400) of either claim 1 or 2, wherein the needle lumen (126, 226, 326) longitudinally extends through the cannula tube (110, 210, 310, 410) and is configured for disposing at least a 5-Fr catheter-and-needle assembly therein.

4. The articulated stiffening cannula (100, 200, 300, 400) of any claim of claims 1-3, wherein the rotatable element is configured to draw the single control line (152, 252, 352, 454) or a first control line (152, 252, 352, 454) of the plurality of control lines (152, 252, 352, 454) into the compound hub (130, 230, 330, 430) upon rotating the rotatable element in a first direction and let the single control line (152, 252, 352, 454) or the first control line (152, 252, 352, 454) out of the compound hub (130, 230, 330, 430) upon rotating the rotatable element in a second, opposite direction,
preferably wherein the first direction of the rotatable element increases the cannula-tube angle and the second direction of the rotatable element decreases the cannula-tube angle,
more preferably wherein the rotatable element is configured to let a second control line (152, 252, 352, 454) of the plurality of control lines (152, 252, 352, 454) out of the compound hub (130, 230, 330, 430) upon rotating the rotatable element in the second direction and draw the second control line (152, 252, 352, 454) into the compound hub (130, 230, 330, 430) upon rotating the rotatable element in the first direction.

5. The articulated stiffening cannula (100, 200, 300, 400) of any claim of claims 1-4, wherein the cannula-tube angle ranges from about 0° to about 35°.

6. The articulated stiffening cannula (100, 200, 300, 400) of claim 1, wherein the cannula tube (110, 210, 310, 410) is stainless steel or nitinol, the cannula tube (110, 210, 310, 410) including a plurality of transverse cutouts (116, 118, 216, 316, 417, 418, 419, 420, 422) filled with a pliable material, the flexible hinge (114, 214, 314, 414) formed at least in part by the pliable material-filled cutouts (116, 118, 216, 316, 417, 418, 419, 420, 422),
preferably wherein the cutouts (116, 118, 216, 316, 417, 418, 419, 420, 422) include a first set of cutouts along a first side of the cannula tube (110, 210, 310, 410) and a second set of cutouts along a second, opposite side of the cannula tube (110, 210, 310, 410), the first set of cutouts configured for compression and the second set of cutouts configured for tension when the cannula tube (110, 210, 310, 410) is articulated at the flexible hinge (114, 214, 314, 414).

7. The articulated stiffening cannula (100, 200, 300, 400) of claim 1 or 6, wherein a proximal-end portion of a first control line (152, 252, 352, 454) of the pair of control lines (152, 252, 352, 454) is coupled to the rotatable hub (132, 232) and a distal-end portion of the first control line (152, 252, 352, 454) is attached to the distal-end portion of the cannula tube (110, 210, 310, 410), the rotatable hub (132, 232) configured to draw the first control line (152, 252, 352, 454) into the compound hub (130, 230, 330, 430) upon rotating the rotatable hub (132, 232) in a first direction and let the first control line (152, 252, 352, 454) out of the compound hub (130, 230, 330, 430) upon rotating the rotatable hub (132, 232) in a second, opposite direction,
preferably wherein the first direction of the rotatable hub (132, 232) increases the cannula-tube angle and the second direction of the rotatable hub (132, 232) decreases the cannula-tube angle,
more preferably wherein a proximal-end portion of a second control line (152, 252, 352, 454) of the pair of control lines (152, 252, 352, 454) is also coupled to the rotatable hub (132, 232) and a distal-end portion of the second control line (152, 252, 352, 454) is also attached to the distal-end portion of the cannula tube (110, 210, 310, 410), the rotatable hub (132, 232) configured to let the second control line (152, 252, 352, 454) out of the compound hub (130, 230, 330, 430) upon rotating the rotatable hub (132, 232) in the second direction and draw the second control line (152, 252, 352, 454) into the compound hub (130, 230, 330, 430) upon rotating the rotatable hub (132, 232) in the first direction.

8. The articulated stiffening cannula (100, 200, 300, 400) of any claim of claims 1, 6 and 7, wherein the rotatable hub (132, 232) is rotatably coupled to the cannula tube (110, 210, 310, 410) and disposed between the fixed hub (134, 234) and a fixed endpiece (136) of the compound hub (130, 230, 330, 430) fixedly coupled to the cannula tube (110, 210, 310, 410), the rotatable hub (132, 232) engaged with the fixed hub (134, 234) in a Hirth or curvic coupling and held against the fixed hub (134, 234) by a compression spring (138) between the rotatable hub (132, 232) and the fixed endpiece (136),
preferably wherein the Hirth or curvic coupling is configured to hold the cannula tube (110, 210, 310, 410) at the chosen cannula-tube angle in combination with the compression spring (138).

9. The articulated stiffening cannula (100, 200, 300, 400) of claim 1, wherein the cannula tube (110, 210, 310, 410) includes a cannula-tube core (218, 318) having one or more tubular layers disposed thereover, the cannula tube (110, 210, 310, 410) including a plurality of transverse cutouts (116, 118, 216, 316, 417, 418, 419, 420, 422) in at least the one or more tubular layers, the flexible hinge (114, 214, 314, 414) formed at least in part by the cutouts (116, 118, 216, 316, 417, 418, 419, 420, 422).

10. The articulated stiffening cannula (100, 200, 300, 400) of claim 1 or 9, wherein a proximal-end portion of the single control line (152, 252, 352, 454) is coupled to the rotatable hub (132, 232) and a distal-end portion of the single control line (152, 252, 352, 454) is attached to the distal-end portion of the cannula tube (110, 210, 310, 410), the rotatable hub (132, 232) configured to draw the single control line (152, 252, 352, 454) into the compound hub (130, 230, 330, 430) upon rotating the rotatable hub (132, 232) in a first direction and let the single control line (152, 252, 352, 454) out of the compound hub (130, 230, 330, 430) upon rotating the rotatable hub (132, 232) in a second, opposite direction,
preferably wherein the first direction of the rotatable hub (132, 232) increases the cannula-tube angle and the second direction of the rotatable hub (132, 232) decreases the cannula-tube angle.

11. The articulated stiffening cannula (100, 200, 300, 400) of any claim of claims 1, 9 and 10, wherein the rotatable hub (132, 232) is rotatably coupled to the cannula tube (110, 210, 310, 410) and disposed in a bore in a proximal-end portion of the fixed hub (134, 234),
preferably wherein the rotatable hub (132, 232) includes a threaded bore concentric with the bore in the fixed hub (134, 234), the threaded bore configured to drive a threaded plug (236) proximally, away from the fixed hub (134, 234), to increase the cannula-tube angle or distally, toward the fixed hub (134, 234) to decrease the cannula-tube angle.

12. An access set, comprising:
a) an introducer sheath (402);
b) the articulated stiffening cannula (100, 200, 300, 400) according to claim 1;
c) a catheter (404); and
d) a needle (406), the stiffening cannula (100, 200, 300, 400) configured to stiffen at least a catheter-and-needle assembly including the needle (406) disposed in the catheter (404) when the catheter-and-needle assembly is disposed in the stiffening cannula (100, 200, 300, 400) in support of a needle throw therefrom at a chosen cannula-tube angle,
preferably wherein the cannula tube (110, 210, 310, 410) includes a needle lumen (126, 226, 326) longitudinally extending through the cannula tube (110, 210, 310, 410) configured for disposing at least a 5-Fr catheter-and-needle assembly therein.

## Patentansprüche

1. Gelenkige Versteifungskanüle (100, 200, 300, 400), umfassend:
ein Kanülenrohr (110, 210, 310, 410), einschließlich eines flexiblen Scharniers (114, 214, 314, 414) entlang einer distalen Länge des Kanülenrohrs (110, 210, 310, 410), wobei das Kanülenrohr (110, 210, 310, 410) so konfiguriert ist, dass es an dem flexiblen Scharnier (114, 214, 314, 414) gelenkig ist, um einen Kanülenrohrwinkel zwischen einem distalen Endabschnitt des Kanülenrohrs (110, 210, 310, 410) und einer proximalen Länge des Kanülenrohrs (110, 210, 310, 410) einzustellen;
eine Verbindungsnabe (130, 230, 330, 430), die etwa um einen proximalen Endabschnitt des Kanülenrohrs (110, 210, 310, 410) angeordnet ist, wobei die Verbindungsnabe (130, 230, 330, 430) eine feste Nabe (134, 234), die fest mit dem Kanülenrohr (110, 210, 310, 410) gekoppelt ist, und eine drehbare Nabe (132, 232) einschließt, die so konfiguriert ist, dass sie sich relativ zu der festen Nabe (134, 234) dreht; und
einen Gelenkmechanismus, der durch mindestens ein drehbares Element der Verbindungsnabe (130, 230, 330, 430) gesteuert wird, wobei der Gelenkmechanismus eine einzelne Steuerleitung (152, 252, 352, 454) oder ein Paar Steuerleitungen (152, 252, 352, 454) einschließt, die sich von der Verbindungsnabe (130, 230, 330, 430) durch das Kanülenrohr (110, 210, 310, 410) über das flexible Scharnier (114, 214, 314, 414) und zum distalen Endabschnitt des Kanülenrohrs (110, 210, 310, 410) zum Einstellen des Kanülenrohrwinkels erstrecken, die Versteifungskanüle (100, 200, 300, 400) konfiguriert ist, um eine Katheteranordnung zu versteifen, die einen Nadelwurf von der Katheteranordnung in einem gewählten Kanülenrohrwinkel trägt,
wobei der Gelenkmechanismus durch mindestens die drehbare Nabe (132, 232) gesteuert wird,
wobei die einzelne Steuerleitung (152, 252, 352, 454) oder jede Steuerleitung (152, 252, 352, 454) des Steuerleitungspaares (152, 252, 352, 454) in einem separaten, dedizierten Steuerleitungslumen (120, 122, 124, 220, 322, 416) angeordnet ist, das sich in Längsrichtung durch das Kanülenrohr (110, 210, 310, 410) neben einem Nadellumen (126, 226, 326) des Kanülenrohrs (110, 210, 310, 410) erstreckt, oder
wobei beide Steuerleitungen (152, 252, 352, 454) des Steuerleitungspaares (152, 252, 352, 454) in einem dedizierten Steuerleitungslumen (120, 122, 124, 220, 322, 416) angeordnet sind, das sich durch das Kanülenrohr (110, 210, 310, 410) neben einem Nadellumen (126, 226, 326) des Kanülenrohrs (110, 210, 310, 410) erstreckt.

2. Gelenkige Versteifungskanüle (100, 200, 300, 400) nach Anspruch 1, wobei das Kanülenrohr (110, 210, 310, 410) eine Vielzahl von Querausschnitten (116, 118, 216, 316, 417, 418, 419, 420, 422) in dem Kanülenrohr (110, 210, 310, 410) einschließt, wobei das flexible Scharnier (114, 214, 314, 414) mindestens teilweise durch die Ausschnitte (116, 118, 216, 316, 417, 418, 419, 420, 422) gebildet ist.

3. Gelenkige Versteifungskanüle (100, 200, 300, 400) entweder nach Anspruch 1 oder 2, wobei sich das Nadellumen (126, 226, 326) in Längsrichtung durch das Kanülenrohr (110, 210, 310, 410) erstreckt und zum Anordnen mindestens einer 5-Fr-Katheter- und Nadelanordnung darin konfiguriert ist.

4. Gelenkige Versteifungskanüle (100, 200, 300, 400) nach einem der Ansprüche 1 bis 3, wobei das drehbare Element konfiguriert ist zum Ziehen der einzelnen Steuerleitung (152, 252, 352, 454) oder einer ersten Steuerleitung (152, 252, 352, 454) der Vielzahl von Steuerleitungen (152, 252, 352, 454) in die Verbindungsnabe (130, 230, 330, 430) beim Drehen des drehbaren Elements in eine erste Richtung und Herauslassen der einzelnen Steuerleitung (152, 252, 352, 454) oder der ersten Steuerleitung (152, 252, 352, 454) aus der Verbindungsnabe (130, 230, 330, 430) beim Drehen des drehbaren Elements in eine zweite, entgegengesetzte Richtung,
vorzugsweise wobei die erste Richtung des drehbaren Elements den Kanülenrohrwinkel vergrößert und die zweite Richtung des drehbaren Elements den Kanülenrohrwinkel verringert,
bevorzugter wobei das drehbare Element konfiguriert ist zum Herauslassen einer zweiten Steuerleitung (152, 252, 352, 454) der Vielzahl von Steuerleitungen (152, 252, 352, 454) aus der Verbindungsnabe (130, 230, 330, 430) beim Drehen des drehbaren Elements in die zweite Richtung und Ziehen der zweiten Steuerleitung (152, 252, 352, 454) in die Verbindungsnabe (130, 230, 330, 430) beim Drehen des drehbaren Elements in die erste Richtung.

5. Gelenkige Versteifungskanüle (100, 200, 300, 400) nach einem der Ansprüche 1 bis 4, wobei der Kanülenrohrwinkel zwischen etwa 0° und etwa 35° liegt.

6. Gelenkige Versteifungskanüle (100, 200, 300, 400) nach Anspruch 1, wobei das Kanülenrohr (110, 210, 310, 410) aus rostfreiem Stahl oder Nitinol besteht, das Kanülenrohr (110, 210, 310, 410) eine Vielzahl von quer verlaufenden Ausschnitten (116, 118, 216, 316, 417, 418, 419, 420, 422) einschließt, die mit einem biegsamen Material gefüllt sind, wobei das flexible Scharnier (114, 214, 314, 414) mindestens teilweise durch die mit biegsamem Material gefüllten Ausschnitte (116, 118, 216, 316, 417, 418, 419, 420, 422) gebildet ist,
vorzugsweise wobei die Ausschnitte (116, 118, 216, 316, 417, 418, 419, 420, 422) einen ersten Satz Ausschnitte entlang einer ersten Seite des Kanülenrohrs (110, 210, 310, 410) und einen zweiten Satz Ausschnitte entlang einer zweiten, gegenüberliegenden Seite des Kanülenrohrs (110, 210, 310, 410) einschließen, wobei der erste Satz an Ausschnitten für Kompression konfiguriert ist und der zweite Satz Ausschnitte für Spannung konfiguriert ist, wenn das Kanülenrohr (110, 210, 310, 410) an dem flexiblen Scharnier (114, 214, 314, 414) gelenkig verbunden ist.

7. Gelenkige Versteifungskanüle (100, 200, 300, 400) nach Anspruch 1 oder 6, wobei ein proximaler Endabschnitt einer ersten Steuerleitung (152, 252, 352, 454) des Paares von Steuerleitungen (152, 252, 352, 454) mit der drehbaren Nabe (132, 232) gekoppelt ist und ein distaler Endabschnitt der ersten Steuerleitung (152, 252, 352, 454) an dem distalen Endabschnitt des Kanülenrohrs (110, 210, 310, 410) befestigt ist, die drehbare Nabe (132, 232) konfiguriert ist zum Ziehen der ersten Steuerleitung (152, 252, 352, 454) in die Verbindungsnabe (130, 230, 330, 430) beim Drehen der drehbaren Nabe (132, 232) in eine erste Richtung und Herauslassen der ersten Steuerleitung (152, 252, 352, 454) aus der Verbindungsnabe (130, 230, 330, 430) beim Drehen der drehbaren Nabe (132, 232) in eine zweite, entgegengesetzte Richtung,
vorzugsweise wobei die erste Richtung der drehbaren Nabe (132, 232) den Kanülenrohrwinkel vergrößert und die zweite Richtung der drehbaren Nabe (132, 232) den Kanülenrohrwinkel verringert,
bevorzugter wobei ein proximaler Endabschnitt einer zweiten Steuerleitung (152, 252, 352, 454) des Paares von Steuerleitungen (152, 252, 352, 454) auch mit der drehbaren Nabe (132, 232) gekoppelt ist und ein distaler Endabschnitt der zweiten Steuerleitung (152, 252, 352, 454) auch an dem distalen Endabschnitt des Kanülenrohrs (110, 210, 310, 410) befestigt ist, die drehbare Nabe (132, 232) konfiguriert ist zum Herauslassen der zweiten Steuerleitung (152, 252, 352, 454) aus der Verbindungsnabe (130, 230, 330, 430) beim Drehen der drehbaren Nabe (132, 232) in die zweite Richtung und Ziehen der zweiten Steuerleitung (152, 252, 352, 454) in die Verbindungsnabe (130, 230, 330, 430) beim Drehen der drehbaren Nabe (132, 232) in die erste Richtung.

8. Gelenkige Versteifungskanüle (100, 200, 300, 400) nach einem der Ansprüche 1, 6 und 7, wobei die drehbare Nabe (132, 232) drehbar mit dem Kanülenrohr (110, 210, 310, 410) gekoppelt und zwischen der festen Nabe (134, 234) und einem festen Endstück (136) der Verbindungsnabe (130, 230, 330, 430) angeordnet ist, das fest mit dem Kanülenrohr (110, 210, 310, 410) gekoppelt ist,
die drehbare Nabe (132, 232) mit der festen Nabe (134, 234) in einer Hirth- oder Curvic-Kopplung eingreift und durch eine Druckfeder (138) zwischen der drehbaren Nabe (132, 232) und dem festen Endstück (136) gegen die feste Nabe (134, 234) gehalten ist,
vorzugsweise wobei die Hirth- oder Curvic-Kopplung so konfiguriert ist, dass sie das Kanülenrohr (110, 210, 310, 410) in Kombination mit der Druckfeder (138) in dem gewählten Kanülenrohrwinkel hält.

9. Gelenkige Versteifungskanüle (100, 200, 300, 400) nach Anspruch 1, wobei das Kanülenrohr (110, 210, 310, 410) einen Kanülenrohrkern (218, 318) einschließt, der eine oder mehrere darüber angeordnete röhrenförmige Schichten aufweist, wobei das Kanülenrohr (110, 210, 310, 410) eine Vielzahl von Querausschnitten (116, 118, 216, 316, 417, 418, 419, 420, 422) in mindestens der einen oder den mehreren röhrenförmigen Schichten einschließt, wobei das flexible Scharnier (114, 214, 314, 414) mindestens teilweise durch die Ausschnitte (116, 118, 216, 316, 417, 418, 419, 420, 422) gebildet ist.

10. Gelenkige Versteifungskanüle (100, 200, 300, 400) nach Anspruch 1 oder 9, wobei ein proximaler Abschnitt der einzelnen Steuerleitung (152, 252, 352, 454) mit der drehbaren Nabe (132, 232) gekoppelt ist und ein distaler Endabschnitt der einzelnen Steuerleitung (152, 252, 352, 454) an dem distalen Endabschnitt des Kanülenrohrs (110, 210, 310, 410) befestigt ist, die drehbare Nabe (132, 232) konfiguriert ist zum Ziehen der einzelnen Steuerleitung (152, 252, 352, 454) in die Verbindungsnabe (130, 230, 330, 430) beim Drehen der drehbaren Nabe (132, 232) in eine erste Richtung und Herauslassen der einzelnen Steuerleitung (152, 252, 352, 454) aus der Verbindungsnabe (130, 230, 330, 430) beim Drehen der drehbaren Nabe (132, 232) in eine zweite, entgegengesetzte Richtung,
vorzugsweise wobei die erste Richtung der drehbaren Nabe (132, 232) den Kanülenrohrwinkel vergrößert und die zweite Richtung der drehbaren Nabe (132, 232) den Kanülenrohrwinkel verringert.

11. Gelenkige Versteifungskanüle (100, 200, 300, 400) nach einem der Ansprüche 1, 9 und 10, wobei die drehbare Nabe (132, 232) drehbar mit dem Kanülenrohr (110, 210, 310, 410) gekoppelt und in einer Bohrung in einem proximalen Endabschnitt der festen Nabe (134, 234) angeordnet ist,
vorzugsweise wobei die drehbare Nabe (132, 232) eine Gewindebohrung einschließt, die konzentrisch zu der Bohrung in der festen Nabe (134, 234) ist, wobei die Gewindebohrung so konfiguriert ist, dass sie einen Gewindestopfen (236) proximal von der festen Nabe (134, 234) weg treibt, um den Kanülenrohrwinkel zu vergrößern, oder distal in Richtung der festen Nabe (134, 234), um den Kanülenrohrwinkel zu verringern.

12. Zugangsset, umfassend:
a) eine Einführhülse (402);
b) die gelenkige Versteifungskanüle (100, 200, 300, 400) nach Anspruch 1;
c) einen Katheter (404); und
d) eine Nadel (406), wobei die Versteifungskanüle (100, 200, 300, 400) konfiguriert ist, um mindestens eine Katheter- und Nadelanordnung zu versteifen, einschließlich der Nadel (406), die in dem Katheter (404) angeordnet ist, wenn die Katheter- und Nadelanordnung in der Versteifungskanüle (100, 200, 300, 400) angeordnet ist, um einen Nadelwurf aus dieser in einem gewählten Kanülenrohrwinkel zu stützen,
vorzugsweise wobei das Kanülenrohr (110, 210, 310, 410) ein Nadellumen (126, 226, 326) einschließt, das sich in Längsrichtung durch das Kanülenrohr (110, 210, 310, 410) erstreckt und zum Anordnen mindestens einer 5-Fr-Katheter- und Nadelanordnung darin konfiguriert ist.

## Revendications

1. Canule de raidissement articulée (100, 200, 300, 400), comprenant :
un tube (110, 210, 310, 410) de canule incluant une charnière flexible (114, 214, 314, 414) le long d'une longueur distale du tube (110, 210, 310, 410) de canule, le tube (110, 210, 310, 410) de canule étant configuré pour s'articuler au niveau de la charnière flexible (114, 214, 314, 414) pour ajuster un angle de tube de canule entre une partie d'extrémité distale du tube (110, 210, 310, 410) de canule et une longueur proximale du tube (110, 210, 310, 410) de canule ;
un raccord composé (130, 230, 330, 430) disposé autour d'une partie d'extrémité proximale du tube (110, 210, 310, 410) de canule, dans laquelle le raccord composé (130, 230, 330, 430) inclut un raccord fixe (134, 234) couplé à demeure au tube (110, 210, 310, 410) de canule et un raccord rotatif (132, 232) configuré pour tourner par rapport au raccord fixe (134, 234) ; et
un mécanisme d'articulation commandé par au moins un élément rotatif du raccord composé (130, 230, 330, 430), le mécanisme d'articulation incluant une ligne de commande unique (152, 252, 352, 454) ou une paire de lignes de commande (152, 252, 352, 454) s'étendant depuis le raccord composé (130, 230, 330, 430), à travers le tube (110, 210, 310, 410) de canule au-delà de la charnière flexible (114, 214, 314, 414), et jusqu'à la partie d'extrémité distale du tube (110, 210, 310, 410) de canule pour ajuster l'angle de tube de canule, la canule de raidissement (100, 200, 300, 400) étant configurée pour raidir un ensemble cathéter en soutien à un retrait d'aiguille de l'ensemble cathéter à un angle de tube de canule choisi,
dans laquelle le mécanisme d'articulation est commandé par au moins un raccord rotatif (132, 232)
dans laquelle la ligne de commande unique (152, 252, 352, 454) ou chaque ligne de commande (152, 252, 352, 454) de la paire de lignes de commande (152, 252, 352, 454) est disposée dans une lumière dédiée séparée (120, 122, 124, 220, 322, 416) de ligne de commande s'étendant longitudinalement à travers le tube (110, 210, 310, 410) de canule à côté d'une lumière (126, 226, 326) d'aiguille du tube (110, 210, 310, 410) de canule, ou
dans laquelle les deux lignes de commande (152, 252, 352, 454) de la paire de lignes de commande (152, 252, 352, 454) sont disposées dans une lumière dédiée (120, 122, 124, 220, 322, 416) de ligne de commande s'étendant à travers le tube (110, 210, 310, 410) de canule à côté d'une lumière (126, 226, 326) d'aiguille du tube (110, 210, 310, 410) de canule.

2. Canule de raidissement articulée (100, 200, 300, 400) selon la revendication 1, dans laquelle le tube (110, 210, 310, 410) de canule inclut une pluralité de découpes transversales (116, 118, 216, 316, 417, 418, 419, 420, 422) dans le tube (110, 210, 310, 410) de canule, la charnière flexible (114, 214, 314, 414) étant formée au moins en partie par les découpes (116, 118, 216, 316, 417, 418, 419, 420, 422).

3. Canule de raidissement articulée (100, 200, 300, 400) selon la revendication 1 ou la revendication 2, dans laquelle la lumière (126, 226, 326) d'aiguille s'étend longitudinalement à travers le tube (110, 210, 310, 410) de canule et est configurée pour y disposer au moins un ensemble cathéter et aiguille 5-Fr.

4. Canule de raidissement articulée (100, 200, 300, 400) selon l'une quelconque des revendications 1 à 3, dans laquelle l'élément rotatif est configuré pour tirer la ligne de commande unique (152, 252, 352, 454) ou une première ligne de commande (152, 252, 352, 454) de la pluralité de lignes de commande (152, 252, 352, 454) dans le raccord composé (130, 230, 330, 430) lors de la rotation de l'élément rotatif dans une première direction et faire sortir la ligne de commande unique (152, 252, 352, 454) ou la première ligne de commande (152, 252, 352, 454) du raccord composé (130, 230, 330, 430) lors de la rotation de l'élément rotatif dans une seconde direction opposée,
préférablement dans laquelle la première direction de l'élément rotatif augmente l'angle de tube de canule et la seconde direction de l'élément rotatif diminue l'angle de tube de canule,
plus préférablement dans laquelle l'élément rotatif est configuré pour faire sortir une seconde ligne de commande (152, 252, 352, 454) de la pluralité de lignes de commande (152, 252, 352, 454) du raccord composé (130, 230, 330, 430) lors de la rotation de l'élément rotatif dans la seconde direction et tirer la seconde ligne de commande (152, 252, 352, 454) dans le raccord composé (130, 230, 330, 430) lors de la rotation de l'élément rotatif dans la première direction.

5. Canule de raidissement articulée (100, 200, 300, 400) selon l'une quelconque des revendications 1 à 4, dans laquelle l'angle de tube de canule va d'environ 0° à environ 35°.

6. Canule de raidissement articulée (100, 200, 300, 400) selon la revendication 1, dans laquelle le tube (110, 210, 310, 410) de canule est de l'acier inoxydable ou du nitinol, le tube (110, 210, 310, 410) de canule incluant une pluralité de découpes transversales (116, 118, 216, 316, 417, 418, 419, 420, 422) remplies d'un matériau malléable, la charnière flexible (114, 214, 314, 414) étant formée au moins en partie par les découpes (116, 118, 216, 316, 417, 418, 419, 420, 422) remplies de matériau malléable,
préférablement dans laquelle les découpes (116, 118, 216, 316, 417, 418, 419, 420, 422) incluent un premier ensemble de découpes le long d'un premier côté du tube (110, 210, 310, 410) de canule et un second ensemble de découpes le long d'un second côté opposé du tube (110, 210, 310, 410) de canule, le premier ensemble de découpes étant configuré pour la compression et le second ensemble de découpes étant configuré pour la tension lorsque le tube (110, 210, 310, 410) de canule s'articule au niveau de la charnière flexible (114, 214, 314, 414).

7. Canule de raidissement articulée (100, 200, 300, 400) selon la revendication 1 ou revendication 6, dans laquelle une partie d'extrémité proximale d'une première ligne de commande (152, 252, 352, 454) de la paire de lignes de commande (152, 252, 352, 454) est couplée au raccord rotatif (132, 232) et une partie d'extrémité distale de la première ligne de commande (152, 252, 352, 454) est fixée à la partie d'extrémité distale du tube (110, 210, 310, 410) de canule, le raccord rotatif (132, 232) est configuré pour tirer la première ligne de commande (152, 252, 352, 454) dans le raccord composé (130, 230, 330, 430) lors de la rotation du raccord rotatif (132, 232) dans une première direction et faire sortir la première ligne de commande (152, 252, 352, 454) du raccord composé (130, 230, 330, 430) lors de la rotation du raccord rotatif (132, 232) dans une seconde direction opposée,
préférablement dans laquelle la première direction du raccord rotatif (132, 232) augmente l'angle de tube de canule et la seconde direction du raccord rotatif (132, 232) diminue l'angle de tube de canule,
plus préférablement dans laquelle une partie d'extrémité proximale d'une seconde ligne de commande (152, 252, 352, 454) de la paire de lignes de commande (152, 252, 352, 454) est également couplée au raccord rotatif (132, 232) et une partie d'extrémité distale de la seconde ligne de commande (152, 252, 352, 454) est également fixée à la partie d'extrémité distale du tube (110, 210, 310, 410) de canule,
le raccord rotatif (132, 232) est configuré pour faire sortir la seconde ligne de commande (152, 252, 352, 454) du raccord composé (130, 230, 330, 430) lors de la rotation du raccord rotatif (132, 232) dans la seconde direction et tirer la seconde ligne de commande (152, 252, 352, 454) dans le raccord composé (130, 230, 330, 430) lors de la rotation du raccord rotatif (132, 232) dans la première direction.

8. Canule de raidissement articulée (100, 200, 300, 400) selon l'une quelconque des revendications 1, 6 et 7, dans laquelle le raccord rotatif (132, 232) est couplé en rotation au tube (110, 210, 310, 410) de canule et disposé entre le raccord fixe (134, 234) et un embout fixe (136) du raccord composé (130, 230, 330, 430) couplé à demeure au tube (110, 210, 310, 410) de canule, le raccord rotatif (132, 232) venant en prise avec le raccord fixe (134, 234) dans un couplage de Hirth ou Curvic et étant maintenu contre le raccord fixe (134, 234) par un ressort de compression (138) entre le raccord rotatif (132, 232) et l'embout fixe (136),
préférablement dans lequel le couplage de Hirth ou Curvic est configuré pour maintenir le tube (110, 210, 310, 410) de canule à l'angle de tube de canule choisi en combinaison avec le ressort de compression (138).

9. Canule de raidissement articulée (100, 200, 300, 400) selon la revendication 1, dans laquelle le tube (110, 210, 310, 410) de canule inclut un noyau (218, 318) de tube de canule présentant une ou plusieurs couches tubulaires disposées par-dessus celui-ci, le tube (110, 210, 310, 410) de canule incluant une pluralité de découpes transversales (116, 118, 216, 316, 417, 418, 419, 420, 422) dans au moins les une ou plusieurs couches tubulaires, la charnière flexible (114, 214, 314, 414) étant formée au moins en partie par les découpes (116, 118, 216, 316, 417, 418, 419, 420, 422).

10. Canule de raidissement articulée (100, 200, 300, 400) selon la revendication 1 ou revendication 9, dans laquelle une partie d'extrémité proximale de la ligne de commande unique (152, 252, 352, 454) est couplée au raccord rotatif (132, 232) et une partie d'extrémité distale de la ligne de commande unique (152, 252, 352, 454) est fixée à la partie d'extrémité distale du tube (110, 210, 310, 410) de canule, le raccord rotatif (132, 232) est configuré pour tirer la ligne de commande unique (152, 252, 352, 454) dans le raccord composé (130, 230, 330, 430) lors de la rotation du raccord rotatif (132, 232) dans une première direction et faire sortir la ligne de commande unique (152, 252, 352, 454) du raccord composé (130, 230, 330, 430) lors de la rotation du raccord rotatif (132, 232) dans une seconde direction opposée,
préférablement dans laquelle la première direction du raccord rotatif (132, 232) augmente l'angle de tube de canule et la seconde direction du raccord rotatif (132, 232) diminue l'angle de tube de canule.

11. Canule de raidissement articulée (100, 200, 300, 400) selon l'une quelconque des revendications 1, 9 et 10, dans laquelle le raccord rotatif (132, 232) est couplé de manière rotative au tube (110, 210, 310, 410) de canule et disposé dans un alésage dans une partie d'extrémité proximale du raccord fixe (134, 234),
préférablement dans laquelle le raccord rotatif (132, 232) inclut un alésage fileté concentrique avec l'alésage dans le raccord fixe (134, 234), l'alésage fileté étant configuré pour entraîner un bouchon fileté (236) de manière proximale, loin du raccord fixe (134, 234), pour augmenter l'angle de tube de canule ou de manière distale, vers le raccord fixe (134, 234) pour diminuer l'angle de tube de canule.

12. Ensemble d'accès, comprenant :
a) une gaine d'introduction (402) ;
b) la canule de raidissement articulée (100, 200, 300, 400) selon la revendication 1 ;
c) un cathéter (404) ; et
d) une aiguille (406), la canule de raidissement (100, 200, 300, 400) étant configurée pour raidir au moins un ensemble cathéter et aiguille incluant l'aiguille (406) disposée dans le cathéter (404) lorsque l'ensemble cathéter et aiguille est disposé dans la canule de raidissement (100, 200, 300, 400) en soutien au retrait d'aiguille de celui-ci à un angle de tube de canule choisi
préférablement dans lequel le tube (110, 210, 310, 410) de canule inclut une lumière (126, 226, 326) d'aiguille s'étendant longitudinalement à travers le tube (110, 210, 310, 410) de canule configurée pour y disposer au moins un ensemble cathéter et aiguille 5-Fr.
